(19) Europäisches Patentamt
European Patent Office
Office européen des brevets

(11) Publication number: **0 331 529 A2**

# (12) EUROPEAN PATENT APPLICATION

(21) Application number: 89302189.9

(22) Date of filing: 03.03.89

(51) Int. Cl.4: C 07 D 237/18
C 07 D 237/22, A 01 N 43/58,
C 07 D 239/42,
C 07 D 239/60, A 01 N 43/54,
C 07 D 239/86, C 07 D 239/56

(30) Priority: 03.03.88 JP 48648/88
05.10.88 JP 249817/88

(43) Date of publication of application:
06.09.89 Bulletin 89/36

(84) Designated Contracting States: DE ES FR GB IT

(71) Applicant: UBE INDUSTRIES, LTD.
12-32, Nishihonmachi 1-chome
Ube-shi, Yamaguchi-ken 755 (JP)

Rikagaku Kenkyusho
Hirosawa 2-1
Wako-shi Saitama-ken (JP)

(72) Inventor: Yoshioka, Hirosuke
c/o Rikagaku Kenkyusho 2-1 Hirosawa
Wako-shi Saitama-ken (JP)

Obata, Tokio Ube Research Laboratory
Ube Industries Ltd. 1978-5 Oaza Kogushi
Ube-shi Yamaguchi-ken (JP)

Fujii, Katsutoshi Ube Research Laboratory
Ube Industries Ltd. 1978-5 Oaza Kogushi
Ube-shi Yamaguchi-ken (JP)

Fukuda, Yasuhisa Ube Research Laboratory
Ube Industries Ltd. 1978-5 Oaza Kogushi
Ube-shi Yamaguchi-ken (JP)

Ooka, Akira Ube Research Laboratory
Ube Industries Ltd. 1978-5 Oaza Kogushi
Ube-shi Yamaguchi-ken (JP)

(74) Representative: Harrison, Michael Robert et al
Urquhart-Dykes & Lord 91 Wimpole Street
London W1M 8AH (GB)

## (54) Diphenyl ether derivatives, process for producing the same and insecticide and acaricide containing the same as active ingredient.

(57) A diphenyl ether derivative of the formula:

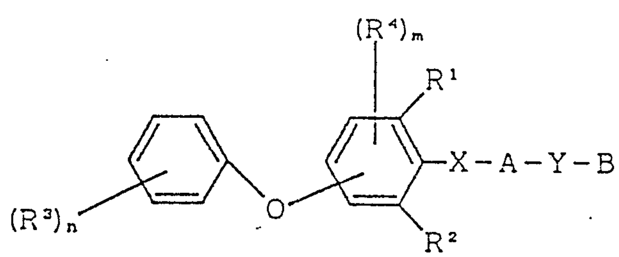

wherein $R^1$, $R^2$, $R^3$ and $R^4$ each independently represent a hydrogen atom, a halogen group, a nitro group, a formyl group, a hydroxyl group, a lower alkyl group which may be unsubstituted or substituted, a lower alkoxy group which may be unsubstituted or substituted, a lower alkylthio group which may be unsubstituted or substituted or lower alkylsulfonyl group which may be unsubstituted or substituted, an aralkyl group which may be unsubstituted or substituted or an aryloxy group which may be unsubstituted or substituted, with proviso that all of $R^1$, $R^2$, $R^3$ and $R^4$ cannot represent a hydrogen atom at the same time; m is an integer of 1 or 2; n is an integer of 1 to 5; X and Y each independently represent oxygen atom, sulfur atom or an imino group which may be unsubstituted or substituted with a lower alkyl group; A represents a straight or branched alkylene group having 2 to 4 carbon atoms; and B represents a heterocyclic group containing at least one nitrogen atom as the hetero atom,
processes for preparing the same and insecticides and acaricides containing the same as active ingredient.

EP 0 331 529 A2

**Description**

**Diphenyl ether derivatives, process for producing the same and insecticide and acaricide containing the same as active ingredient**

## BACKGROUND OF THE INVENTION

This invention relates to diphenyl ether derivatives, processes for preparing the same and insecticides and acaricides containing the same as active ingredient.

Heretofore, a large number of pyridazinone derivatives have been known as compounds having insecticidal, acaricidal and fungicidal activities.

For example, 5-phenylalkoxy- or 5-phenylalkylthiopyridazinone derivatives (Japanese Unexamined Patent Publications Nos. 152878/1983, 98064/1984, 4173/1985, 32774/1985, 54370/1985), and 5-aralkyloxy- or 5-aralkylthiopyridazinone derivatives (Japanese Unexamined Patent Publication No. 268672/1986), etc. have been disclosed. Moreover, compounds of the following formula:

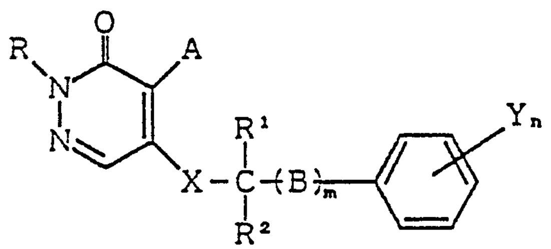

wherein R is an alkyl group having 1 to 6 carbon atoms; $R^1$ and $R^2$ are each independently hydrogen atom, a lower alkyl group, a lower haloalkyl group, etc.; X is oxygen atom or sulfur atom; Y is hydrogen atom, an alkyl group having 1 to 6 carbon atoms, a cycloalkyl group; A is an alkyl group having 1 to 6 carbon atoms; B is $-CR^3=CR^4-$ group, $-CR^3R^4O-$ group (wherein $R^3$ and $R^4$ represent hydrogen atom or an alkyl group having 1 to 3 carbon atoms); m is 0 or 1; n is an integer of 1 to 5,
are disclosed in Japanese Unexamined Patent Publication No. 130275/1986, and also compounds of the following formula:

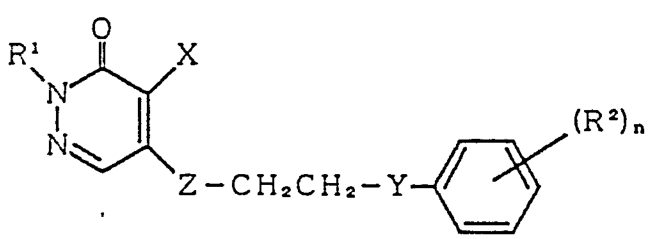

wherein $R^1$ is a lower alkyl group; $R^2$ is hydrogen atom, a lower alkyl group, a lower alkylthio group, a halogen atom, an alkoxycarbonyl group; X is a halogen atom; Y and Z are each independently oxygen atom or sulfur atom; n is 1 or 2,
are disclosed in Japanese Unexamined Patent Publication No. 238274/1987.

The above known compounds have insecticidal, acaricidal, fungicidal activities, and are also effective against, for example, injurious insects such as green rice leafhopper, twenty eight spotted ladybird, etc. and Kanzawa spider mite, and also against injurious diseases such as cucumber downy mildew, cucumber powdery mildew, wheat leaf rust, rice blast, etc.

However, although said compounds have insecticidal and acaricidal activities, their effects cannot be said to be satisfactory.

The present inventors have made intensive studies in order to obtain compounds having further excellent insecticidal and acaricidal activities than the above known compounds, and consequently successfully synthesized novel diphenyl ether derivatives, and further found that said derivatives have remarkably improved insecticidal and acaricidal activities, to accomplish the present invention.

## SUMMARY OF THE INVENTION

The present invention provides diphenyl ether derivatives represented by the formula (I):

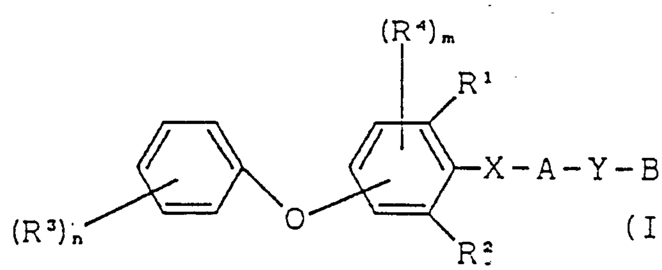

(I)

wherein $R^1$, $R^2$, $R^3$ and $R^4$ each independently represent a hydrogen atom, a halogen atom, a nitro group, a formyl group, a hydroxyl group, a lower alkyl group which may be unsubstituted or substituted, a lower alkoxy group which may be unsubstituted or substituted, a lower alkylthio group which may be unsubstituted or substituted or lower alkylsulfonyl group which may be unsubstituted or substituted, an aralkyl group which may be unsubstituted or substituted or an aryloxy group which may be unsubstituted or substituted, with proviso that all of $R^1$, $R^2$, $R^3$ and $^4$ cannot represent a hydrogen atom at the same time; m is an integer of 1 or 2; n is an integer of 1 to 5; X and Y each independently represent oxygen atom, sulfur atom or an imino group which may be unsubstituted or substituted with a lower alkyl group; A represents a straight or branched alkylene group having 2 to 4 carbon atoms; and B represents a heterocyclic group containing at least one nitrogen atom as the hetero atom,
processes for producing the same and insecticides and acaricides containing the same as the active ingredient.

## DESCRIPTION OF THE PREFERRED EMBODIMENTS

In the above formula (I), the lower alkyl represented by $R^1$, $R^2$, $R^3$ or $R^4$ refers to a straight or branched alkyl group having 1 to 5 carbon atoms. Examples of such alkyl group may include methyl, ethyl, propyl, isopropyl, butyl, isobutyl, sec-butyl, tert-butyl, amyl, isoamyl, sec-amyl, sec-isoamyl (1,2-dimethylpropyl), tert-amyl (1,1-dimethylpropyl) groups and the like. Similarly, the lower alkoxy group refers to a straight or branched alkoxy group having 1 to 5 carbon atoms. Examples of such alkoxy group may include methoxy, ethoxy, propoxy, isopropoxy, butoxy, isobutoxy, sec-butoxy, t-butoxy, amyloxy groups and the like. Similarly, the lower alkylthio group refers to a straight or branched alkylthio group having 1 to 5 carbon atoms. Examples of such alkylthio group may include methylthio, ethylthio, propylthio, isopropylthio, butylthio, isobutylthio, sec-butylthio, t-butylthio, amylthio groups and the like. Similarly, the lower alkylsulfonyl group refers to a straight or branched alkylsulfonyl group having 1 to 5 carbon atoms. Examples of such alkylsulfonyl group may include methylsulfonyl, ethylsulfonyl, propylsulfonyl, isopropylsulfonyl, butylsulfonyl, isobutylsulfonyl, sec-butylsulfonyl, t-butylsulfonyl, amylsulfonyl groups and the like. Similarly, as the halogen group, fluorine atom, chlorine atom, bromine atom may be included.

The above lower alkyl group, lower alkoxy group, lower alkylthio group and lower alkylsulfonyl group may be substituted with suitable substituents. As such substituent, there may be included halogen atoms, lower alkenyl groups, cycloalkyl groups having 3 to 6 carbon atoms, lower alkylthio groups, lower alkoxy groups, lower alkoxyalkoxy groups, lower alkenyloxy groups, lower alkynyloxy groups, aralkyloxy groups, etc.

As the substituted lower alkyl group, trifluoromethyl, 2-chloroethyl, 2,2,2-trifluoroethyl, ethoxymethyl, 2-methoxyethyl, 1-ethoxyethyl, 2-ethoxyethyl, 2-allyloxyethyl, 2-propargyloxyethyl groups, etc. may be included. As the substituted lower alkoxy group, trifluoromethoxy, cyclopropylmethoxy, 2-chloroethoxy, 2,2,2-trifluoroethoxy, 2-ethoxyethoxy, 2-benzyloxyethoxy, 2-ethylthioethoxy groups, etc. may be included. As the substituted lower alkylthio group, trifluoromethylthio, 2-chloroethylthio, 2,2,2-trifluoroethylthio, 2-ethoxy ethylthio, 2-benzyloxyethylthio, 2-ethylthioethylthio groups, etc. may be included. As the substituted lower alkylsulfonyl group, trifluoromethylsulfonyl, 2-chloroethylsulfonyl, 2,2,2-trifluoroethylsulfonyl, 2-ethoxyethylsulfonyl, 2-benzyloxyethylsulfonyl, 2-ethylthioethylsulfonyl groups, etc. may be included.

In the above formula (I), the substituted or unsubstituted aralkyl group represented by $R^1$, $R^2$, $R^3$ or $R^4$ may include benzyl, p-chlorobenzyl, p-fluorobenzyl, 3,4-dichlorobenzyl, 1-phenethyl, 2-phenethyl, etc, and similarly the substituted or unsubstituted aryloxy group represented by $R^1$, $R^2$, $R^3$ or $R^4$ may include phenoxy, p-chlorophenoxy, p-fluorophenoxy, p-trifluoromethylphenoxy, p-methylphenoxy, 2,4-dichlorophenoxy, 3,4-dichlorophenoxy, etc.

As $R^1$ and $R^2$, methyl group and chlorine atom are preferred, while as $R^3$, methyl group, 2-fluoroethyl group,

fluorine atom, bromine atom, chlorine atom, ethoxy, t-butoxy, difluoromethoxy, trifluoromethoxy, 2-methoxyethyl, 2-ethoxyethoxy, methylthio and formyl groups, etc. are preferred, and as the substituted position, the 4-position is preferred. As $R^4$, hydrogen atom, methyl group and chlorine atom are preferred.

As the lower alkyl-substituted or unsubstituted imino group of Y, methyl-substituted imino group or unsubstituted imino group are preferred.

In the above formula (I), the heterocyclic group represented by B may be any group which contains at least one nitrogen atom as the hetero atom, and examples thereof may include those represented by the following formulae (B1) - (B17).

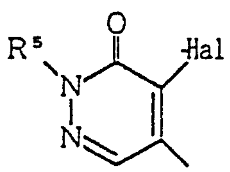

(B 1)

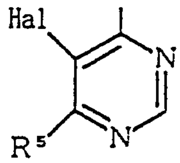

(B 2)

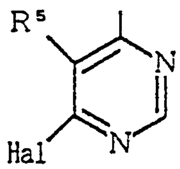

(B 3)

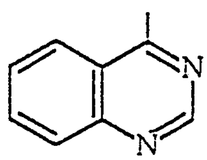

(B 4)

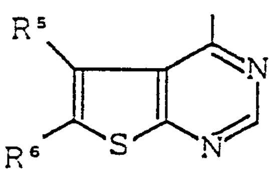

(B 5)

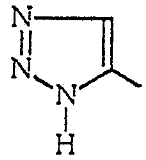

(B6)

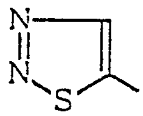

(B7)

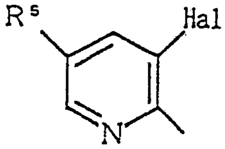

(B8)

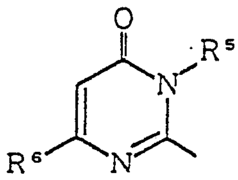

(B9)

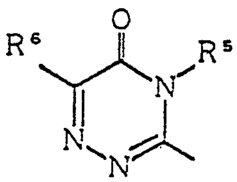

(B10)

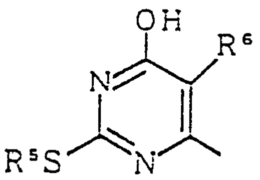

(B11)

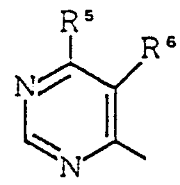

(B12)

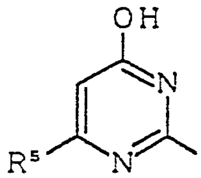

(B13)

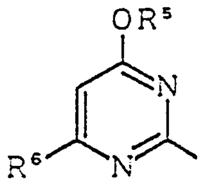

(B14)

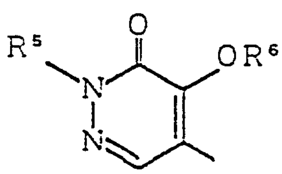

(B15)

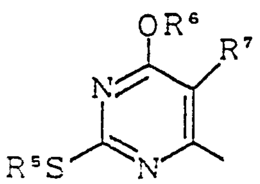

(B16)

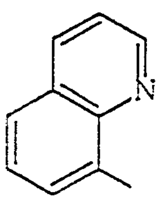

(B17)

wherein $R^5$, $R^6$ and $R^7$ represent a lower alkyl group having 1 to 3 carbon atoms as described above for $R^1$, $R^2$

$R^3$ and $R^4$; Hal represents a halogen atom as described above.

Among the diphenyl ether derivatives of this invention, 4-substituted derivatives represented by the formula (I'''') are preferred:

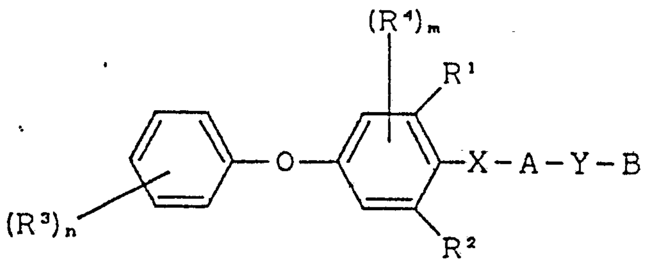

(I'''')

The compounds (I) of the present invention can be prepared easily according to the methods known per se.

Preparation method (1)

A compound of the formula (II):

B-Hal (II)

wherein B is the same as defined above; Hal represents a halogen atom is allowed to react with a compound of the formula (III):

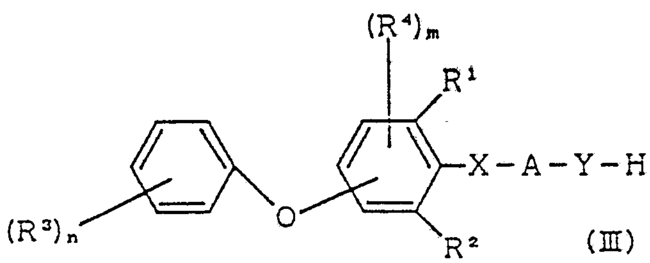

(III)

wherein $R^1$, $R^2$, $R^3$, $R^4$, m, n, X, Y and A are the same as defined above.

Preparation method (2)

A compound of the formula (IV):

B-Y-H (IV)

wherein B and Y are the same as defined above is allowed to react with a compound of the formula (V):

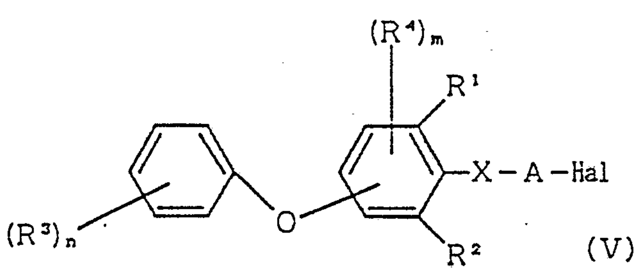

(V)

wherein $R^1$, $R^2$, $R^3$, $R^4$, m, n, X, Y and A are the same ad defined above.

As is apparent from the above reactions, in both of the cases of the preparation methods (1) and (2), as the result of the reaction, hydrogen halide (HHal) is eliminated. Therefore, in order to carry out the reaction smoothly by trapping this, it is preferable to carry out the reaction in the presence of a base.

As the base, organic bases such a triethylamine, pyridine, N,N-dimethylanilline, alkoxides of alkali metals

such as sodium methoxide, sodium ethoxide, inorganic bases such as sodium hydride, sodium amide, sodium hydroxide, potassium hydroxide, sodium carbonate, sodium hydrogen carbonate, potassium carbonate may be employed.

The reaction is generally carried out in the presence of a solvent. The solvent is not particularly limited, provided that it does not participate in the present reaction, and may include, for example, chlorinated or non-chlorinated aromatic, aliphatic, alicyclic hydrocarbons such as benzene, toluene, xylene, methyl naphthalene, petroleum ether, ligroin, hexane, chlorobenzene, dichlorobenzene, methylene chloride, chloroform, dichloroethane, trichloroethylene, cyclohexane; ethers such as diethyl ether, ethylene glycol dimethyl ether, tetrahydrofuran, dioxane; ketones such as acetone, methyl ethyl ketone, methyl isobutyl ketone; alcohols such as methanol, ethanol, ethylene glycol or hydrates thereof; amides such as N,N-dimethylformamide, N,N-dimethylacetamide, 1,3-dimethyl-2-imidazolidinone, dimethylsulfoxide, water and mixtures of the above solvents, etc.

However, when an inorganic base such as sodium hydroxide, potassium hydroxide, etc. is used as the base as described above, it is preferable to use water or a mixture of the above solvent with water in dissolving these bases.

On the other hand, when a mixture of water and non-aqueous solvent is used, it is preferable to add a small amount of a phase transfer catalyst, and examples of such phase transfer catalyst may include quaternary ammonium salts such as tetramethylammonium chloride, triethylbenzylammonium chloride, tetra-n-butylammonium bromide or tri-n-octylmethylammonium chloride, phosphonium salts such as tetra-n-butylphosphonium bromide, crown ethers such as dicyclohexano-18-crown-6 or cryptands, etc.

The reaction temperature is not particularly limited, but generally from 0 °C to the boiling point of the solvent employed, and it is preferable to heat the reaction mixture in order to shorten the reaction time.

Preparation method (3)

When $R^3$ is a substituted or unsubstituted alkoxy group in the compound (I) of the present invention, the compound can be also prepared by allowing a compound of the formula (I′):

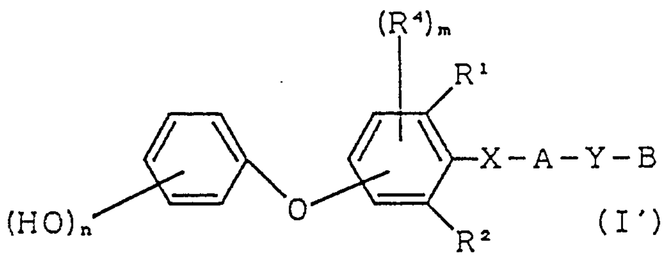

(I′)

wherein $R^1$, $R^2$, $R^4$, m, n, X, Y, A and B are the same as defined above to react with a compound of the formula (IV) or (VII):

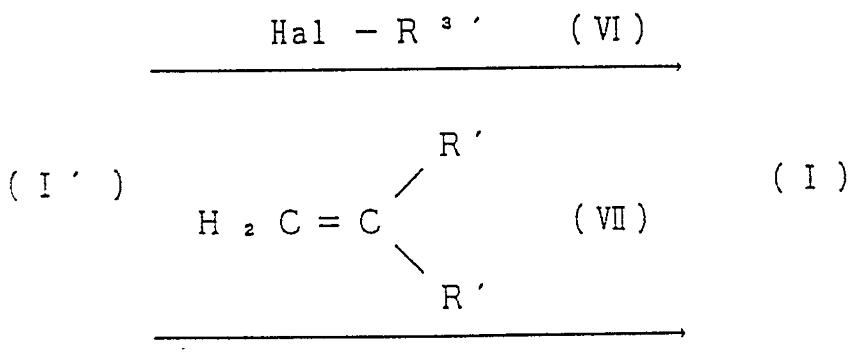

wherein Hal is the same as defined above; $R^{3'}$ represents a substituted or unsubstituted alkyl group having 1 to 5 carbon atoms, and R′ represents a lower alkyl group.

Of the above reaction schemes, the reaction with the compound (VI) is a reaction in which hydrogen halide (HHal) is eliminated similarly as in the preparation methods (1) and (2), and the same base, solvent and conditions as in the preparation methods (1) and (2) can be suitably applied, but in the reaction with the compound (VII), which is an addition reaction of an olefin (VII), it is preferable to add an acid catalyst for

promoting the reaction.

As the acid catalyst, sulfuric acid, phosphoric acid, polyphosphoric acid, hydrogen chloride, zinc chloride, titanium tetrachloride, aluminum chloride, etc. may be employed.

Preparation method (4)

In the compound (I) of the present invention wherein $R^3$ is difluoromethyl group, the compound can be also prepared by allowing a compound of the formula (I″):

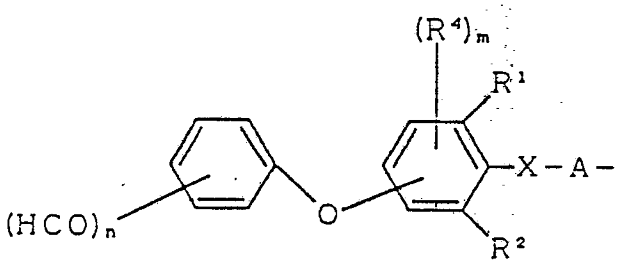

(I″)

wherein $R^1$, $R^2$, $R^4$, m, n, X, Y, A and B are the same as defined above,
to react with diethylaminosulfur trifluoride (DAST).

The reaction is generally carried out in the presence of a solvent, but the reaction can be also carried out by heating the compound of the formula (I″) and DAST without any solvent.

The solvent is not particularly limited, provided that is does not participate in the present reaction, and the same solvent and conditions as in the preparation methods (1) and (2) can be suitably applied.

Preparation method (5)

In the compound (I) of the present invention wherein $R^3$ is trifluoromethoxy group, the compound can be also prepared by allowing a compound of the formula (I‴):

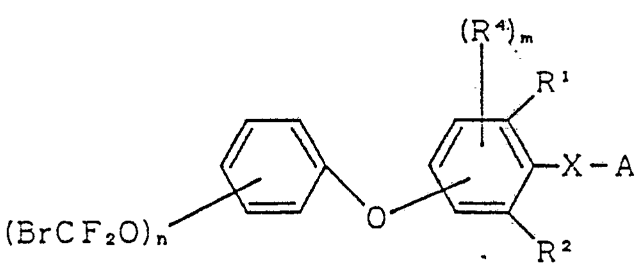

(I‴)

wherein $R^1$, $R^2$, $R^4$, m, n, X, Y, A and B are the same as defined above,
to react with $AgBF_4$.

The reaction is generally carried out in the presence of a solvent, but it can be also carried out by heating the compound of the formula (I‴) and $AgBF_4$ without any solvent.

The solvent is not particularly limited, provided that it does not participate in the present reaction, and the same solvent and conditions as in the preparation methods (1) and (2) can bu suitably applied.

The compounds of the formula (II) to be used as the starting material in the above preparation methods are known compounds (see, for example, Japanese Unexamined Patent Publications Nos. 175173/1982 and 10561/1986), and the compounds of the formulae (III), (IV) and (V) can be easily prepared by the methods known per se shown below.

The compound of the formula (IV) wherein Y is oxygen atom or sulfur atom can be prepared by treating the compound of the above formula (II) with a compound of the following formula (VIII):

NaYH (VIII)

wherein Y is the same as defined above.

The compound of the formula (V) can be prepared by allowing a compound of the formula (IX):

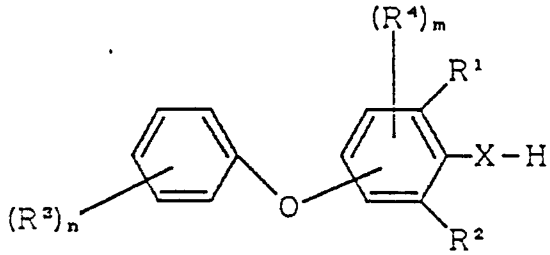

(IX)

wherein $R^1$, $R^2$, $R^3$, $R^4$ and X are the same as defined above, to react with a compound of the formula (X):

Hal-A-Hal (X)

wherein A and Hal are the same as defined above,

and by treating the compound (V) thus obtained with a compound of the formula (XI) or (VIII):

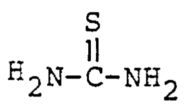 or NaSH

(XI) (VIII),

the compound of the formula (III) wherein Y is sulfur atom can be prepared.

Also, the compounds of the formula (III), wherein Y is oxygen atom, can be easily prepared according to the following method.

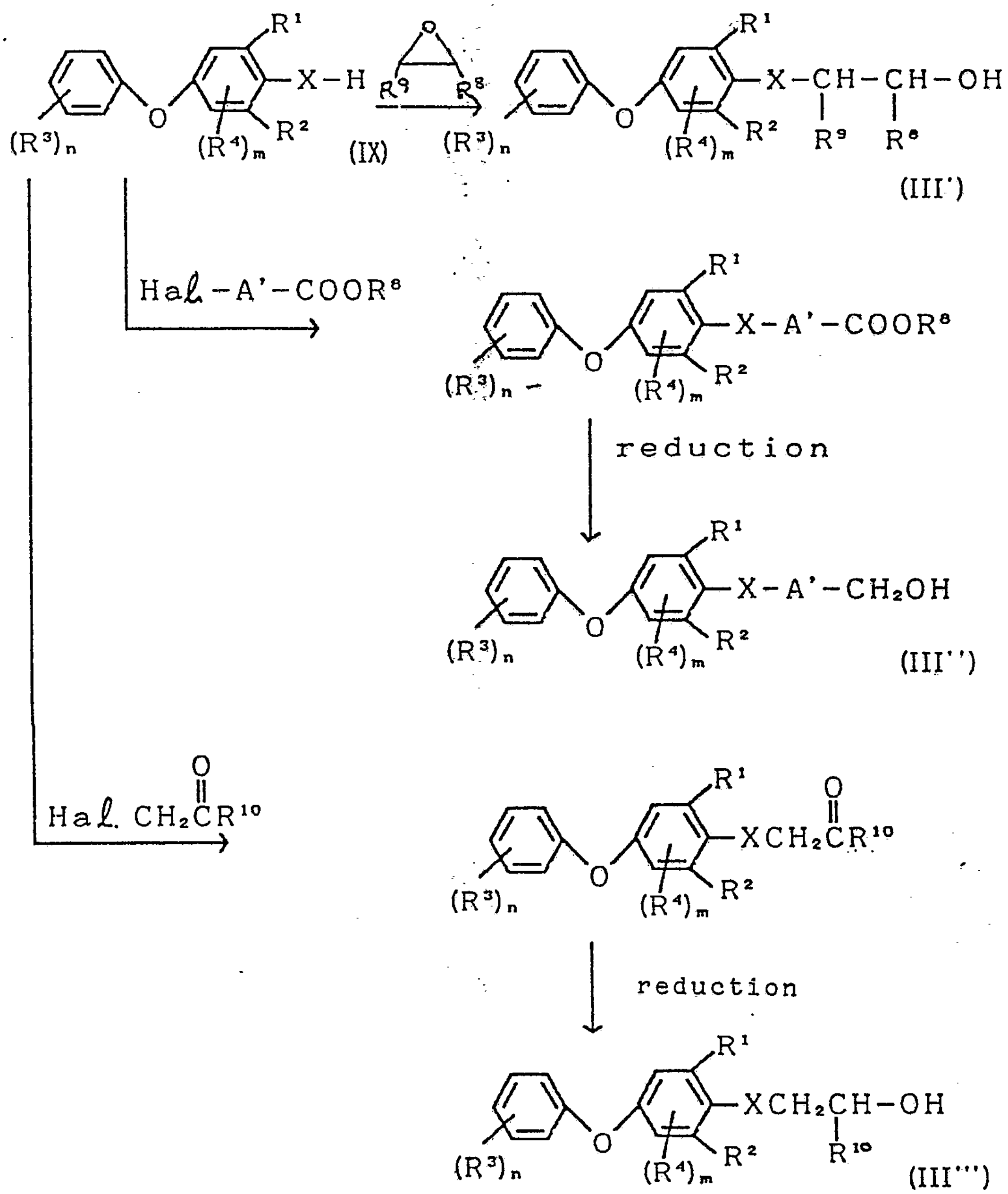


wherein $R^1$, $R^2$, $R^3$, $R^4$, X, m and n are the same as defined above, $R^8$ represents lower alkyl group or hydrogen atom, $R^9$ and $R^{10}$ represent a lower alkyl group and A′ represents straight and branched alkylene group having 1 to 4 carbon atoms.

The compounds of the formula (III), wherein Y is a lower alkyl-substituted or unsubstituted imino group, can be easily prepared according to the following methods.

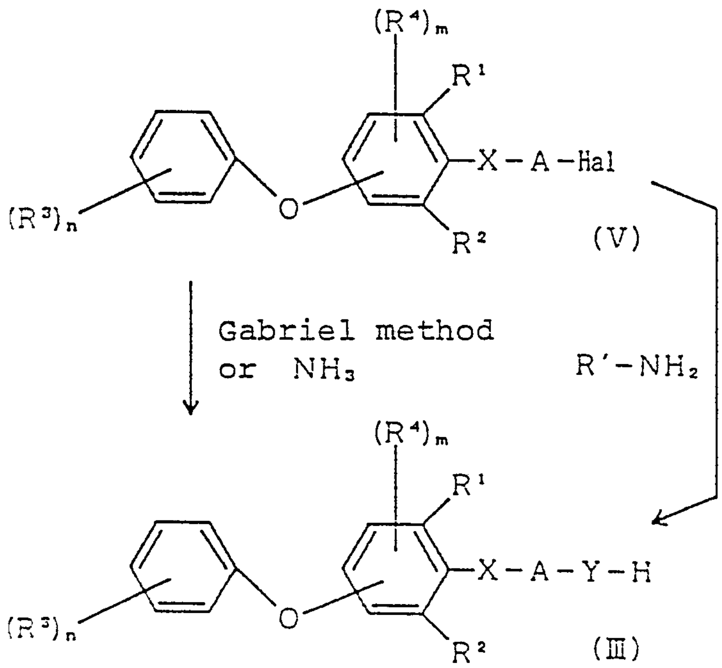


(V)

(III)

wherein $R^1$, $R^2$, $R^3$, $R^4$, m, n, X, A and Hal are the same as defined above, R′ represents a lower alkyl group, and Y represents a lower alkyl-substituted or unsubstituted imino group.

The desired compound (I) obtainable according to the above method can be appropriately purified according to known means such a recrystallization, various chromatographies, etc.

The compounds of the present invention exhibit excellent effects against agricultural and horticultural injurious insects, including Hemiptera such as planthoppers, leafhopper, aphids, whiteflies, etc.; Lepidoptera such as pyralid cabbage armyworms, diamondback moth, leaf folding worms, pyralid moths, common cabbage worms, etc.; Coleoptera such as weevils, leaf beatles, etc.; and otherwise Acarina such as citrus red mite, two-spotted spider mite, etc. Also, they are extremely effective for control of hygienically injurious insects such as flies, mosquitos, cockroaches, etc. and otherwise also effective against injurious insects to stored grains, etc.

Further, the compounds of the present invention have also activities against root-knot nematodes, pine wood nematode, bulb mites. The compounds of the present invention are also effective for injurious diseases for agriculture and horticulture, and have activities against, for example, rice blast, barley powdery mildew, and otherwise cucumber downy mildew, gray mold, etc.

Thus, the compounds of the present invention have extremely wide uses and application fields, having high activities, and can be provided for practical application in various preparation forms.

The insecticide and acaricide of the present invention comprises one or several kinds of the compounds of the formula (I) as the active ingredient. Although the compound of the formula (I) itself may be used, but it is generally used by formulating conventional carriers, surfactants, dispersing agents or auxiliary agents, etc. in conventional manner to be prepared into such compositions as powder, wettable powder, emulsion, fine granule, granule, aqueous or oily suspension, aerosol, etc. before use.

Suitable carriers may include, for example, solid carriers such as talc, bentonite, clay, kaolin, diatomaceous earth, white carbon, vermiculite, slaked lime, siliceous sand, ammonium sulfate, urea, etc.; liquid carriers, including hydrocarbons such as kerosine, mineral oil, etc., aromatic hydrocarbons such as benzene, toluene, xylene, etc., chlorinated hydrocarbons such as chloroform, carbon tetrachloride, etc., ethers such as dioxane, tetrahydrofuran, etc., ketones such as acetone, cyclohexanone, isophorone, etc., esters such as ethyl acetate, ethylene glycol acetate, dibutyl maleate, etc., alcohols such as methanol, n-hexanol, ethylene glycol, etc., polar solvents such as dimethylformamide, dimethylsulfoxide, etc., or water and others. Also, as the gas carrier, air, nitrogen, carbon dioxide, Freon, etc. may be used and jetted as a mixture.

As the surfactant, dispersing agent for effecting improvement of attachment, absorbance of the present agent onto animals and vegetables, as well as improvement of performance of the chemicals such as dispersion, emulsification, spreading, etc., there may be employed, for example, alcohol sulfate esters, alkylsulfonic acid salts, lignin sulfonic acid salts, polyoxyethylene glycol ethers, etc.

Further, for improvement of the properties of the preparation, as the auxiliary agent, for example, carboxymethyl cellulose, polyethylene glycol, gum arabic, etc. may be employed.

The above carrier, surfactant, dispersing agent and auxiliary agent may be used each individually or as a

combination depending on the respective purposes.

In case where the compound of the present invention is formed into a preparation, the active ingredient concentration may be generally 1 to 50 % by weight for emulsion, generally 0.3 to 25% by weight for powder, generally 1 to 90 % by weight for wettable powder, generally 0.5 to 5 % by weight for granules, generally 0.5 to 5 % by weight for oil agent, and generally 0.1 to 5 % by weight for aerosol.

These preparations may be diluted to suitable concentrations and sprayed on vegetable stalks and leaves, into soil or on a water surface of paddle field, or directly applied, thus being provided for various uses depending on the respective purposes.

The present invention is described in more detail below by referring to Examples, but these Examples are not limitative of the scope of the present invention at all.

Example 1

Synthesis of
2-tert-butyl-4-chloro-5-{2-[2,6-dimethyl-4-(4-chlorophenoxy)phenoxy]ethylthio}-3(2H)pyridazinone
(Compound No. 2)

To a solution of 2.2 g of 2-tert-butyl-4-chloro-5-mecapto-3(2H)pyridazinone and 3.6 g of 2-[2,6-dimethyl--4-(4-chlorophenoxy)phenoxy]ethylbromide dissolved in 50 ml of N,N-dimethylformamide was added 1.4 g of anhydrous potassium carbonate, and the mixture was stirred at 40 to 60 °C for 4 hours.

After completion of the reaction, the reaction mixture was poured into water and the oily product separated was extracted with toluene. The extract was washed with water, dried over anhydrous sodium sulfate and then toluene was evaporated under reduced pressure.

The oily product obtained was isolated by column chromatography (Waco Gel C-200, eluted with toluene : ethyl acetate = 9 : 1), and then recrystallized from n-hexane to give 3.6 g of the title product as colorless sandy crystals.
m.p. 125 - 127 °C

Example 2

Synthesis of
2-tert-butyl-4-chloro-5-{2-[2,6-dimethyl-4-(4-methylphenoxy)phenoxy]ethylthio}-3(2H)pyridazinone
(Compound No. 6)

To a solution of 2.2 g of 2-tert-butyl-4,5-dichloro-3(2H)-pyridazinone and 3.2 g of 2-[2,6-dimethyl-4-(4-methylphenoxy)phenoxy]ethylmercaptane dissolved in 50 ml of methanol, with cooling to 5 °C or lower under stirring, was added dropwise a solution of 0.6 g of sodium methoxide dissolved in 5 ml of methanol.

After the dropwise addition, the mixture was stirred at room temperature for 1 hour to complete the reaction.

Subsequently, methanol was evaporated under reduced pressure, water was added to the residue and the oily product separated was extracted with ethyl acetate. The extract was washed with water, dried over anhydrous sodium sulfate, and then ethyl acetate was evaporated under reduced presure. The oily product obtained was isolated by column chromatography (Waco Gel C-200, eluted with toluene : ethyl acetate = 9 : 1) and then recrystallized from n-hexane to give 3.5 g of the title product as colorless sandy crystals.
m.p. 131 - 133 °C

Example 3

Synthesis of
2-tert-butyl-4-chloro-5-{2-[2,6-dimethyl-4-(4-fluorophenoxy)phenoxy]ethylamino}-3(2H)pyridazinone
(Compound No. 7)

To a solution of 1.1 g of 2-tert-butyl-4,5-dichloro3-(2H)-pyridazinone and 1.5 g of 2-[2,6-dimethyl-4-(4-fluorophenoxy)phenoxy]ethylamine dissolved in a solvent mixture of 20 ml of dioxane and 10 ml of water was added 0.7 g of potassium carbonate, and then the mixture was heated under reflux under stirring for 8 hours. After completion of the reaction, water was added and the mixture was extracted with toluene. The extract was washed with water, dried over anhydrous sodium sulfate and then toluene was evaporated under reduced pressure. The oil product obtained was isolated by column chromatography (Waco Gel C-200, eluted with toluene : ethyl acetate = 3 : 1) to give 0.7 g of the title product as pale yellow oil product.
$n_D^{17.6}$ 1.5721

Example 4

Synthesis of 5-chloro-6-ethyl-4-{2-[2,6-dimethyl-4-(4-fluorophenoxy)phenoxy]ethylamino}pyrimidine
(Compound No. 10)

A solution of 0.9 g of 4,5-dichloro-6-ethylpyrimidine, 1.5 g of 2-[2,6-dimethyl-4-(4-fluorophenoxy)phenoxy]-ethylamine and 0.6 g of triethylamine dissolved in 30ml of toluene was heated under reflux under stirring for 8 hours. After completion of the reaction, triethylamine hydrochloride was filtered off, and toluene was

evaporated off under reduced pressure. The oily product obtained was isolated by column chromatography (Waco Gel C-200, eluted with toluene : ethyl acetate = 3 : 1) to obtain 0.8 g of the title product as pale yellow oily product.
$n_D^{17.6}$ 1.5800

Example 5

Synthesis of 2-tert-butyl-4-chloro-5-{2-[2,6-dimethyl-4-(4-trifluoromethylphenoxy)phenoxy]ethylthio}-3(2H)-pyridazinone (Compound No. 51)

To a solution of 1.5 g of 2-tert-butyl-4-chloro-5-mercapto-3(2H)pyridazinone and 2.7 g of 2-[2,6-dimethyl--4-(4-trifluoromethylphenoxy)phenoxy]ethylbromide dissolved in 20 ml of N,N-dimethylformamide was added 1.0 g of potassium carbonate, and then the mixture was stirred at room temperature for 6 hours. After completion of the reaction, the reaction mixture was poured into water and extracted with toluene. The extract was washed with water, dried over anhydrous sodium sulfate and then toluene was evaporated under reduced pressure. The oily product obtained was isolated by column chromatography (Waco Gel C-200, eluted with toluene : ethyl acetate = 20 : 1) to obtain 3.2 g of the title product as pale yellow oily product.

Example 6

Synthesis of 2-tert-butyl-4-chloro-5-{2-[2-chloro-6-methyl-4-(4-chlorophenoxy)phenoxy]ethylamino}-3(2H)-pyridazinone (Compound No. 103)

To a solution of 1.5 g of 2-tert-butyl-4-dichloro-3(2H)pyridazinone and 2.2 g of 2-[2-chloro-6-methyl-4-(4-chlorophenoxy)phenoxy]ethylamine dissolved in a solvent mixture of 20 ml of dioxane and 10 ml of water was added 1.0 g of potassium carbonate, and then the mixture was heated under reflux under stirring for 8 hours. After completion of the reaction, water was added and the mixture was extracted with toluene. The extract was washed with water, dried over anhydrous magnesium sulfate and then toluene was evaporated off under reduced pressure. The oily product obtained was isolated by column chromatography (Waco Gel C-200, eluted with toluene : ethyl acetate = 3 : 1) to obtain 2.0 g of the title product as colorless sandy crystals.
m.p. 91 - 94 °C

Example 7

Synthesis of 2-tert-butyl-4-chloro-5-{2-[2-chloro-6-methyl-4-(4-trifluoromethoxyphenoxy)phenoxy]ethylthio}-3-(2H)pyridazinone (Compound No. 23)

A mixture of 0.75 g of 2-tert-butyl-4-chloro-5-{2-[2-chloro-6-methyl-4-(4-bromodifluoromethoxyphenoxy)phenoxy]ethylthio}-3(2H)pyridazinone (Compound No. 21) and 0.75 g of $AgBF_4$ was heated under reflux in 50 ml of toluene for 20 hours.

After completion of the reaction, the reaction mixture was poured into water and extracted with toluene. The extract was washed with water, dried over anhydrous sodium sulfate and then toluene was evaporated off under reduced pressure. The oily product obtained was isolated by column chromatography (Waco Gel C-200, eluted with toluene : ethyl acetate = 20 : 1) to obtain 0.5 g of the title product as colorless oily product.

Example 8

Synthesis of 2-tert-butyl-4-chloro-5-{2-[2-chloro-6-methyl-4-(4-i-propoxyphenoxy)phenoxy]ethylthio}-3(2H)-pyridazinone (Compound No. 77)

To a solution of 2.2 g of 2-tert-butyl-4-chloro-5-mercapto-3(2H)pyridazinone and 4 g of 2-[2-chloro-6-methyl-4-(4-i-propoxyphenoxy)phenoxy]ethylbromide dissolved in 50 ml of N,N-dimethylformamide was added 2 g of anhydrous potassium carbonate, and the mixture was stirred at room temperature for 8 hours. After completion of the reaction, the reaction mixture was poured into water and extracted with toluene. The extract was washed with water, dried over anhydrous sodium sulfate and then toluene was evaporated off under reduced pressure.

The oil product obtained was isolated by column chromatography (Waco Gel C-200, eluted with toluene : ethyl acetate = 100 : 1) to obtain 2 g of the title compound as colorless oily product.

Example 9

Synthesis of 2-tert-butyl-4-chloro-5-{2-[2-chloro-6-methyl-4-(4-t-butoxyphenoxy)phenoxy]ethylthio}-3(2H)-pyridazinone (Compound No. 104)

(1) Synthesis of 2-tert-butyl-4-chloro-5-{2-[2-chloro-6-methyl-4-(4-hydroxyphenoxy)phenoxy]ethylthio}-3(2H)-pyridazinone

To a solution of 1.2 g of 2-tert-butyl-4-chloro-5- mercapto-3(2H)pyridazinone and 2.0 g of 2-[2-chloro-6-methyl-4-(4-hydroxyphenoxy)phenoxy]ethylbromide dissolved in 30 ml of N,N-dimethylformamide was added 1.2 g of anhydrous potassium carbonate, and the mixture was stirred at room temperature for 8 hours. After completion of the reaction, the reaction mixture was poured into water and extracted with toluene. The extract was washed with water, dried over anhydrous sodium sulfate and toluene was evaporated off under reduced pressure. To the oily product obtained was added hexane to be crystallized and then the crystals were collected by filtration. Further, the crystals were recrystallized from toluene-hexane to give 1.6 g of the title product as colorless powdery crystals.

m.p. 175 - 178 °C

(2) Synthesis of 2-tert-butyl-4-chloro-5-{2-[2-chloro-6-methyl-4-(4-t-butoxyphenoxy)phenoxy]ethylthio}-3(2H)-pyridazinone (Compound No. 104)

Into a solution of 1 g of 2-tert-butyl-4-chloro-5-{2-[2-chloro-6-methyl-4-(4-hydroxyphenoxy)phenoxy]ethylthio}-3(2H)pyridazinone dissolved in 25 ml of dichloromethane was blown 1 g of isobutylene. To the mixture was added 0.05 g of conc. sulfuric acid, and the mixture was stirred at room temperature for 8 hours, and was further left to stand for 2 days. The reaction mixture was poured into water, made alkaline with sodium hydrogen carbonate and then the mixture was extracted with toluene. The extract was washed with water, dried over anhydrous sodium sulfate and then solvent was evaporated under reduced pressure.

The oil product obtained was isolated by column chromatography (Waco Gel C-200, eluted with toluene : ethyl acetate = 70 : 1) to obtain 0.18 g of the title product as colorless oily product.

Example 10

Synthesis of 2-tert-butyl-4-chloro-5-{2-[2-chloro-6-methyl-4-(4-formylphenoxy)phenoxy]ethylthio}-3(2H)-pyridazinone (Compound No. 114)

To a solution of 0.5 g of 2-tert-butyl-4-chloro-5-mercapto-3(2H)pyridazinone and 0.9 g of 2-[2-chloro-6-methyl-4-(4-formylphenoxy)phenoxy]ethyliodide dissolved in 20 ml of N,N-dimethylformamide was added 0.4 g of anhydrous potassium carbonate, and the mixture was stirred at room temperature for 8 hours. After completion of the reaction, the reaction mixture was poured into water and extracted with toluene. The extract was washed with water, dried over anhydrous sodium sulfate and toluene was evaporated off under reduced pressure.

The oily product obtained was isolated by column chromatography (Waco Gel C-200, eluted with toluene : ethyl acetate = 50 : 1) to obtain 0.4 g of the title product as colorless oily product.

Example 11

Synthesis of 2-tert-butyl-4-chloro-5-{2-[2-chloro-6-methyl-4-(4-difluoromethylphenoxy)phenoxy]ethylthio}-3(2H)pyridazinone (Compound No. 106)

To 0.3 g of 2-tert-butyl-4-chloro-5-{2-[2-chloro-6-methyl-4-(4-formylphenoxy)phenoxy]ethylthio}-3(2H)-pyridazinone dissolved in 25 ml of dichloromethane was added, at room temperature under stirring, 0.2 g of diethylaminosulfur trifluoride. After the reaction for 8 hours, the reaction mixture was poured into water and extracted with toluene. The extract was washed with water, dried over anhydrous sodium sulfate and then solvent was evaporated under reduced pressure.

The oily product obtained was isolated by column chromatography (Waco Gel C-200, eluted with toluene : ethyl acetate = 60 : 1) to obtain 0.3 of the title product as colorless oily product.

Example 12

By carrying out the treatment in the same manner as described in Examples 1 - 11, the compounds shown under the compounds Nos. 1, 3 - 5, 11, 15 -22, 24 - 31, 33 -35, 37, 39, 41 - 43, 45, 47, 49, 52, 53, 55, 57, 59, 61 -65, 67, 69, 71, 73 - 75, 79 - 81, 83, 85 - 87, 89, 90, 92, 94, 96 - 98, 100 - 102, 105, 108, 110 - 113, 115 -137 as shown in Table 1A and Table 1B were obtained.

Also, the compound of Comparative example 1 as shown in Table 1A and Table 1B was synthesized.

Table 1A

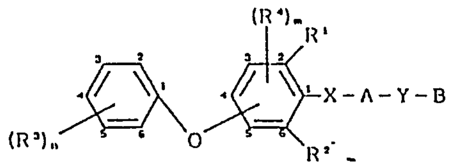

| Compound No. | $R^1$ | $R^2$ | $(R^3)_n$ | $(R^4)_m$ | X | A | Y | B | Substitution position of 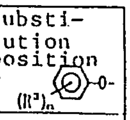 |
|---|---|---|---|---|---|---|---|---|---|
| 1 | H | $CH_3$ | 4-Cℓ | H | O | $-CH_2CH_2-$ | S | 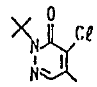 | 4 |
| 2 | $CH_3$ | ″ | ″ | ″ | ″ | ″ | ″ | ″ | ″ |
| 3 | ″ | ″ | H | ″ | ″ | ″ | ″ | ″ | ″ |
| 4 | ″ | Cℓ | 4-Cℓ | ″ | ″ | ″ | ″ | ″ | ″ |
| 5 | ″ | $CH_3$ | 4-F | ″ | ″ | ″ | ″ | ″ | ″ |
| 6 | ″ | ″ | 4-$CH_3$ | ″ | ″ | ″ | ″ | ″ | ″ |
| 7 | ″ | ″ | 4-F | ″ | ″ | ″ | NH | ″ | ″ |
| 8 | ″ | Cℓ | 4-Cℓ | ″ | ″ | ″ | S | 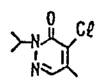 | ″ |
| 9 | ″ | ″ | ″ | ″ | ″ | ″ | ″ | 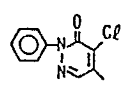 | ″ |
| 10 | ″ | $CH_3$ | 4-F | ″ | ″ | ″ | NH | 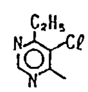 | ″ |
| 11 | ″ | ″ | ″ | ″ | ″ | ″ | ″ | 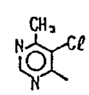 | ″ |
| 12 | ″ | Cℓ | 4-Cℓ | ″ | ″ | ″ | S | 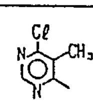 | ″ |
| 13 | ″ | ″ | ″ | ″ | ″ | ″ | ″ | 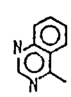 | ″ |
| 14 | ″ | ″ | ″ | ″ | ″ | ″ | ″ | 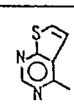 | ″ |

Table 1A (cont'd)

| Compound No. | $R^1$ | $R^2$ | $(R^3)_n$ | $(R^4)_m$ | X | A | Y | B | Substitution position of 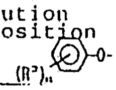 |
|---|---|---|---|---|---|---|---|---|---|
| 15 | $CH_3$ | $CH_3$ | 4-F | H | O | $-CH_2CH_2-$ | S | 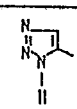 | 4 |
| 16 | 〃 | 〃 | 〃 | 〃 | 〃 | 〃 | 〃 | 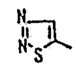 | 〃 |
| 17 | Cl | Cl | 4-Cl | 〃 | 〃 | 〃 | 〃 | 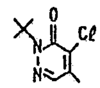 | 〃 |
| 18 | $CH_3$ | 〃 | 4-F | 〃 | 〃 | 〃 | 〃 | 〃 | 〃 |
| 19 | 〃 | 〃 | $4-CH_2CH_2OC_2H_5$ | 〃 | 〃 | 〃 | 〃 | 〃 | 〃 |
| 20 | 〃 | 〃 | $4-OC_2H_5$ | 〃 | 〃 | 〃 | 〃 | 〃 | 〃 |
| 21 | 〃 | 〃 | $4-OCBrF_2$ | 〃 | 〃 | 〃 | 〃 | 〃 | 〃 |
| 22 | 〃 | 〃 | $3,4-Cl_2$ | 〃 | 〃 | 〃 | 〃 | 〃 | 〃 |
| 23 | 〃 | 〃 | $4-OCF_3$ | 〃 | 〃 | 〃 | 〃 | 〃 | 〃 |
| 24 | 〃 | 〃 | $4-OC_4H_9-t$ | 〃 | 〃 | 〃 | 〃 | 〃 | 〃 |
| 25 | 〃 | 〃 | $4-OCHF_2$ | 〃 | 〃 | 〃 | 〃 | 〃 | 〃 |
| 26 | Cl | 〃 | H | 〃 | 〃 | 〃 | 〃 | 〃 | 〃 |
| 27 | $CH_3$ | 〃 | $4-OC_2H_5$ | 〃 | 〃 | 〃 | 〃 | 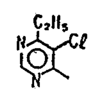 | 〃 |
| 28 | 〃 | 〃 | 4-F | 〃 | 〃 | 〃 | 〃 | 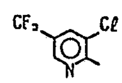 | 〃 |
| 29 | 〃 | 〃 | $2,4-Cl_2$ | 〃 | 〃 | 〃 | 〃 | 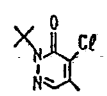 | 〃 |
| 30 | 〃 | 〃 | 4-Cl | 〃 | 〃 | 〃 | O | 〃 | 〃 |
| 31 | 〃 | 〃 | 〃 | 〃 | 〃 | 〃 | 〃 | 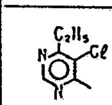 | 〃 |
| 32 | 〃 | 〃 | $4-OC_3F_7-n$ | 〃 | 〃 | 〃 | S | 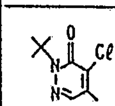 | 〃 |
| 33 | 〃 | $CH_3$ | H | 〃 | 〃 | 〃 | NH | 〃 | 〃 |

Table 1A (cont'd)

| Compound No. | $R^1$ | $R^2$ | $(R^3)_n$ | $(R^4)_m$ | X | A | Y | B | Substitution position of 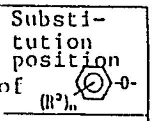 |
|---|---|---|---|---|---|---|---|---|---|
| 34 | $CH_3$ | $CH_3$ | H | H | O | $-CH_2CH_2-$ | NH | 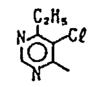 | 4 |
| 35 | 〃 | 〃 | 〃 | 〃 | 〃 | 〃 | 〃 | 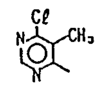 | 〃 |
| 36 | 〃 | Cl | $4-OCH_2CF_3$ | 〃 | 〃 | 〃 | S | 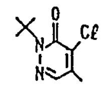 | 〃 |
| 37 | 〃 | 〃 | 3-Cl | 〃 | 〃 | 〃 | 〃 | 〃 | 〃 |
| 38 | 〃 | 〃 | $4-OC_2F_5$ | 〃 | 〃 | 〃 | 〃 | 〃 | 〃 |
| 39 | 〃 | 〃 | 2-Cl | 〃 | 〃 | 〃 | 〃 | 〃 | 〃 |
| 40 | Cl | 〃 | $2.3.5.6-F_4.4-CF_3$ | 〃 | 〃 | 〃 | 〃 | 〃 | 〃 |
| 41 | $CH_3$ | 〃 | H | 〃 | 〃 | 〃 | 〃 | 〃 | 〃 |
| 42 | 〃 | 〃 | $4-CH_3$ | 〃 | 〃 | 〃 | 〃 | 〃 | 〃 |
| 43 | 〃 | 〃 | $4-OC_2H_5$ | 〃 | 〃 | 〃 | NH | 〃 | 〃 |
| 44 | 〃 | 〃 | $4-CH_2OC_2H_5$ | 〃 | 〃 | 〃 | S | 〃 | 〃 |
| 45 | 〃 | 〃 | 4-Br | 〃 | 〃 | 〃 | 〃 | 〃 | 〃 |
| 46 | 〃 | 〃 | $4-CH_2CH_2OCH_3$ | 〃 | 〃 | 〃 | 〃 | 〃 | 〃 |
| 47 | $C_2H_5$ | 〃 | 4-Cl | 〃 | 〃 | 〃 | 〃 | 〃 | 〃 |
| 48 | $CH_3$ | 〃 | $4-CH_2OC_3H_7$-n | 〃 | 〃 | 〃 | 〃 | 〃 | 〃 |
| 49 | $C_2H_5$ | Br | 4-Br | 〃 | 〃 | 〃 | 〃 | 〃 | 〃 |
| 50 | $CH_3$ | Cl | $4-CH_2CH_2OC_3H_7$-n | 〃 | 〃 | 〃 | 〃 | 〃 | 〃 |
| 51 | 〃 | $CH_3$ | $4-CF_3$ | 〃 | 〃 | 〃 | 〃 | 〃 | 〃 |
| 52 | 〃 | Cl | 2-Cl | 〃 | 〃 | 〃 | NH | 〃 | 〃 |
| 53 | 〃 | 〃 | 〃 | 〃 | 〃 | 〃 | 〃 | 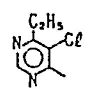 | 〃 |
| 54 | 〃 | 〃 | $4-CH_2CH_2OC_3H_7$-i | 〃 | 〃 | 〃 | S | 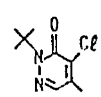 | 〃 |
| 55 | $C_2H_5$ | 〃 | 4-Cl | 〃 | 〃 | 〃 | NH | 〃 | 〃 |
| 56 | $CH_3$ | 〃 | $4-CH_2OC_3H_7$-i | 〃 | 〃 | 〃 | S | 〃 | 〃 |

Table 1A (cont'd)

| Compound No. | $R^1$ | $R^2$ | $(R^3)_n$ | $(R^4)_m$ | X | A | Y | B | Substitution position of $(R^3)_n$ 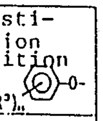 -O- |
|---|---|---|---|---|---|---|---|---|---|
| 57 | $C_2H_5$ | Br | 4-Br | H | O | $-CH_2CH_2-$ | NH | 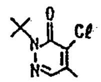 | 4 |
| 58 | $CH_3$ | Cℓ | $4-CH_2OC_4H_9-n$ | 〃 | 〃 | 〃 | S | 〃 | 〃 |
| 59 | 〃 | 〃 | $4-CH_3$ | 〃 | 〃 | 〃 | NH | 〃 | 〃 |
| 60 | 〃 | 〃 | $4-CH_2OCH_2-$ 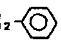 | 〃 | 〃 | 〃 | S | 〃 | 〃 |
| 61 | 〃 | 〃 | 4-F | 〃 | 〃 | 〃 | NH | 〃 | 〃 |
| 62 | 〃 | 〃 | $4-CH_3$ | 〃 | 〃 | 〃 | 〃 | 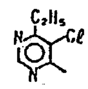 | 〃 |
| 63 | 〃 | 〃 | 4-F | 〃 | 〃 | 〃 | 〃 | 〃 | 〃 |
| 64 | 〃 | 〃 | $4-C_3H_7-n$ | 〃 | 〃 | 〃 | S | 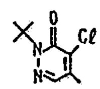 | 〃 |
| 65 | 〃 | 〃 | $4-C_2H_5$ | 〃 | 〃 | 〃 | 〃 | 〃 | 〃 |
| 66 | 〃 | 〃 | $4-CH_2OCF_3$ | 〃 | 〃 | 〃 | 〃 | 〃 | 〃 |
| 67 | 〃 | 〃 | $2-CH_3, 4-C\ell$ | 〃 | 〃 | 〃 | 〃 | 〃 | 〃 |
| 68 | F | F | 4-Cℓ | 〃 | 〃 | 〃 | 〃 | 〃 | 〃 |
| 69 | $CH_3$ | Cℓ | $4-C_3H_7-n$ | 〃 | 〃 | 〃 | NH | 〃 | 〃 |
| 70 | 〃 | F | 4-Cℓ | 〃 | 〃 | 〃 | S | 〃 | 〃 |
| 71 | 〃 | Cℓ | $4-C_2H_5$ | 〃 | 〃 | 〃 | NH | 〃 | 〃 |
| 72 | 〃 | 〃 | $4-CH_2CH_2OC_4H_9-n$ | 〃 | 〃 | 〃 | S | 〃 | 〃 |
| 73 | 〃 | 〃 | $2,4-C\ell_2$ | 〃 | 〃 | 〃 | NH | 〃 | 〃 |
| 74 | 〃 | 〃 | $2-CH_3, 4-C\ell$ | 〃 | 〃 | 〃 | 〃 | 〃 | 〃 |
| 75 | 〃 | $-CH_2-$ 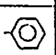 | 4-Cℓ | 〃 | 〃 | 〃 | S | 〃 | 〃 |
| 76 | 〃 | Cℓ | $4-CH_2CH_2OCH_2CF_3$ | 〃 | 〃 | 〃 | 〃 | 〃 | 〃 |
| 77 | 〃 | 〃 | $4-OC_3H_7-i$ | 〃 | 〃 | 〃 | 〃 | 〃 | 〃 |
| 78 | 〃 | 〃 | $4-CH_2CH_2OC_2F_5$ | 〃 | 〃 | 〃 | 〃 | 〃 | 〃 |
| 79 | 〃 | 〃 | 4-F | 〃 | 〃 | 〃 | 〃 | 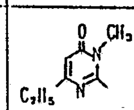 | 〃 |
| 80 | 〃 | 〃 | 〃 | 〃 | 〃 | 〃 | 〃 | 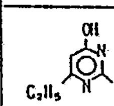 | 〃 |

Table 1A (cont'd)

| Compound No. | $R^1$ | $R^2$ | $(R^3)_n$ | $(R^4)_m$ | X | A | Y | B | Substitution position of 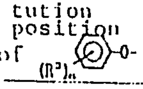 |
|---|---|---|---|---|---|---|---|---|---|
| 81 | $CH_3$ | Cl | 4-F | H | O | $-CH_2CH_2-$ | S | 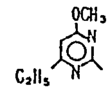 | 4 |
| 82 | 〃 | 〃 | $4-CH_2CH_2OCH_2-$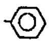 | 〃 | 〃 | 〃 | 〃 | 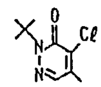 | 〃 |
| 83 | 〃 | 〃 | $4-OCH_3$ | 〃 | 〃 | 〃 | 〃 | 〃 | 〃 |
| 84 | 〃 | $CH_3$ | $4-CH_2OCH_3$ | 〃 | 〃 | 〃 | 〃 | 〃 | 〃 |
| 85 | 〃 | Cl | 4-Cl | 〃 | 〃 | $-CH_2CH(CH_3)-$ | O | 〃 | 〃 |
| 86 | 〃 | 〃 | 〃 | 〃 | 〃 | 〃 | S | 〃 | 〃 |
| 87 | 〃 | 〃 | $4-CF_3$ | 〃 | 〃 | $-CH_2CH_2-$ | 〃 | 〃 | 〃 |
| 88 | 〃 | 〃 | $4-CH_2OC_2H_5$ | 〃 | 〃 | 〃 | 〃 | 〃 | 〃 |
| 89 | 〃 | 〃 | $4-CH_3$ | 〃 | 〃 | 〃 | 〃 | 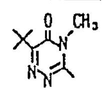 | 〃 |
| 90 | 〃 | 〃 | 4-Cl | 〃 | 〃 | 〃 | O | 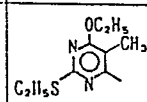 | 〃 |
| 91 | Cl | 〃 | $4-CH_2OCH_3$ | 〃 | 〃 | 〃 | S | 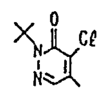 | 〃 |
| 92 | $CH_3$ | 〃 | 4-Cl | 〃 | 〃 | 〃 | O | 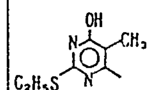 | 〃 |
| 93 | Cl | 〃 | $4-CH_2OC_2H_5$ | 〃 | 〃 | 〃 | S | 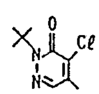 | 〃 |
| 94 | $CH_3$ | 〃 | $4-OC_3H_7-n$ | 〃 | 〃 | 〃 | 〃 | 〃 | 〃 |
| 95 | Cl | 〃 | $4-CH_2CH_2OCH_3$ | 〃 | 〃 | 〃 | 〃 | 〃 | 〃 |
| 96 | $CH_3$ | 〃 | $4-OCH_2CH_2OC_2H_5$ | 〃 | 〃 | 〃 | 〃 | 〃 | 〃 |
| 97 | 〃 | 〃 | 4-Cl | 〃 | 〃 | $-(CH_2)_4-$ | 〃 | 〃 | 〃 |
| 98 | 〃 | 〃 | $4-OCH_2-$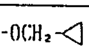 | 〃 | 〃 | $-CH_2CH_2-$ | 〃 | 〃 | 〃 |
| 99 | Cl | 〃 | $4-CH_2CH_2OC_2H_5$ | 〃 | 〃 | 〃 | 〃 | 〃 | 〃 |
| 100 | $CH_3$ | 〃 | $4-OCH_2CH_2F$ | 〃 | 〃 | 〃 | 〃 | 〃 | 〃 |

Table 1A (cont'd)

| Compound No. | R¹ | R² | $(R^3)_n$ | $(R^4)_m$ | X | A | Y | B | Substitution position of 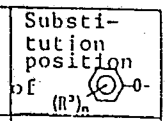 |
|---|---|---|---|---|---|---|---|---|---|
| 101 | $CH_3$ | $CH_3$ | 4-$CH_3$ | H | O | $-CH_2CH_2-$ | NH | 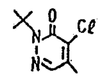 | 4 |
| 102 | 〃 | Cℓ | 4-$CH_2OCH_3$ | 〃 | 〃 | 〃 | S | 〃 | 〃 |
| 103 | 〃 | 〃 | 4-Cℓ | 〃 | 〃 | 〃 | NH | 〃 | 〃 |
| 104 | 〃 | 〃 | 4-$OC_4H_9$-t | 〃 | 〃 | 〃 | S | 〃 | 〃 |
| 105 | 〃 | 〃 | 4-$OCH_2CH_2OCF_3$ | 〃 | 〃 | 〃 | 〃 | 〃 | 〃 |
| 106 | 〃 | 〃 | 4-$CHF_2$ | 〃 | 〃 | 〃 | 〃 | 〃 | 〃 |
| 107 | 〃 | 〃 | 4-Cℓ | 〃 | 〃 | 〃 | 〃 | 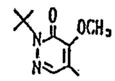 | 〃 |
| 108 | 〃 | 〃 | 4-$OCH_2CH_2OCH_2CH_2OC_2H_5$ | 〃 | 〃 | 〃 | 〃 | 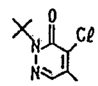 | 〃 |
| 109 | 〃 | 〃 | 4-Cℓ | 〃 | 〃 | 〃 | 〃 | 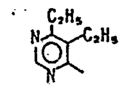 | 〃 |
| 110 | 〃 | 〃 | 4-O-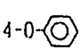 | 〃 | 〃 | 〃 | 〃 | 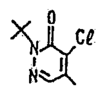 | 〃 |
| 111 | 〃 | 〃 | 4-$SCH_3$ | 〃 | 〃 | 〃 | 〃 | 〃 | 〃 |
| 112 | 〃 | 〃 | 4-$SO_2CH_3$ | 〃 | 〃 | 〃 | 〃 | 〃 | 〃 |
| 113 | 〃 | 〃 | 4-$NO_2$ | 〃 | 〃 | 〃 | 〃 | 〃 | 〃 |
| 114 | 〃 | 〃 | 4-CHO | 〃 | 〃 | 〃 | 〃 | 〃 | 〃 |
| 115 | 〃 | 〃 | 4-$C_2H_5$ | 〃 | 〃 | 〃 | NH | 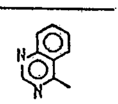 | 〃 |
| 116 | 〃 | 〃 | 4-F | 〃 | 〃 | 〃 | 〃 | 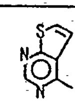 | 〃 |
| 117 | 〃 | 〃 | 〃 | 〃 | 〃 | 〃 | O | 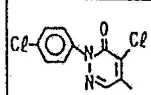 | 〃 |
| 118 | 〃 | 〃 | 4-$CH_3$ | 〃 | 〃 | 〃 | S | 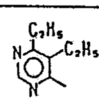 | 〃 |
| 119 | 〃 | 〃 | 4-$OC_3H_7$-i | 〃 | 〃 | 〃 | NH | 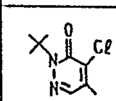 | 〃 |

Table 1A (cont'd)

| Compound No. | $R^1$ | $R^2$ | $(R^3)_n$ | $(R^4)_m$ | X | A | Y | B | Substitution position of 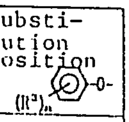 |
|---|---|---|---|---|---|---|---|---|---|
| 120 | $CH_3$ | Cℓ | 4-$CH_3$ | H | O | -$CH_2CH_2$- | S | 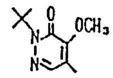 | 4 |
| 121 | Cℓ | 〃 | 4-$CF_3$ | 3-Cℓ | 〃 | 〃 | 〃 | 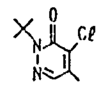 | 〃 |
| 122 | 〃 | 〃 | 〃 | 〃 | 〃 | 〃 | NH | 〃 | 〃 |
| 123 | $CH_3$ | 〃 | 4-$CH_2CH_2OCH_3$ | H | 〃 | 〃 | O | 〃 | 〃 |
| 124 | Cℓ | 〃 | 4-Cℓ | 〃 | 〃 | 〃 | NH | 〃 | 〃 |
| 125 | $CH_3$ | 〃 | 4-$SCH_3$ | 〃 | 〃 | 〃 | S | 〃 | 〃 |
| 126 | 〃 | 〃 | 〃 | 〃 | 〃 | 〃 | NH | 〃 | 〃 |
| 127 | 〃 | 〃 | 3-Cℓ | 〃 | 〃 | 〃 | 〃 | 〃 | 〃 |
| 128 | 〃 | 〃 | 4-Cℓ | 〃 | 〃 | 〃 | -N(CH_3)- | 〃 | 〃 |
| 129 | 〃 | 〃 | 〃 | 〃 | 〃 | -$CH_2$-CH($CH_3$)- | NH | 〃 | 〃 |
| 130 | 〃 | H | 〃 | 〃 | 〃 | 〃 | 〃 | 〃 | 〃 |
| 131 | Cℓ | 〃 | H | 4-Cℓ | 〃 | -$CH_2CH_2$- | S | 〃 | 5 |
| 132 | 〃 | Cℓ | 4-Cℓ | 〃 | 〃 | 〃 | 〃 | 〃 | 〃 |
| 133 | $CH_3$ | 〃 | 4-$CH_2CH_2OCH_3$ | H | 〃 | 〃 | NH | 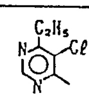 | 4 |
| 134 | 〃 | 〃 | 〃 | 〃 | 〃 | 〃 | O | 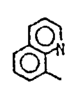 | 〃 |
| 135 | 〃 | Br | 4-Cℓ | 3-Br | 〃 | 〃 | S | 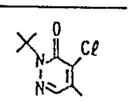 | 〃 |
| 136 | H | H | 4-$CF_3$ | 3,5-$(CH_3)_2$ | 〃 | 〃 | 〃 | 〃 | 〃 |
| 137 | $CH_3$ | $NO_2$ | H | 3-Cℓ | 〃 | 〃 | 〃 | 〃 | 〃 |
| 138 | 〃 | Cℓ | 3,5-$C\ell_2$,2,4-$F_2$ | H | 〃 | 〃 | 〃 | 〃 | 〃 |
| 139 | 〃 | 〃 | 〃 | 〃 | 〃 | 〃 | NH | 〃 | 〃 |
| Comparative example 1 | H | H | H | 〃 | 〃 | 〃 | S | 〃 | 〃 |

Table 1B

| Compound No. | Physical property mp (℃) 又は $n_D$ | PMR δ ppm, CDCℓ₃ |
|---|---|---|
| 1 | mp 162−165 | |
| 2 | mp 125−127 | |
| 3 | mp 107−108 | |
| 4 | mp 47−53 | |
| 5 | mp 117−118 | |
| 6 | mp 131−133 | |
| 7 | $n_D^{17.6}$ 1.5721 | |
| 10 | $n_D^{17.6}$ 1.5800 | |
| 11 | mp 70−72 | |
| 15 | $n_D^{17.6}$ 1.5822 | |
| 16 | mp 79−81 | |
| 17 | $n_D^{20.8}$ 1.5995 | |
| 18 | mp 109−110 | |
| 19 | | 1.21 (3H, t) 1.65 (9H, s) 2.27 (3H, s) 2.88 (2H, t) 3.43−3.67 (6H, m) 4.16 (2H, t) 6.72−7.20 (6H, m) 7.79 (1H, s) |
| 20 | $n_D^{21.4}$ 1.5895 | |
| 21 | | 1.66 (9H, s) 2.30 (3H, s) 3.48 (2H, t) 4.18 (2H, t) 6.77−7.20 (6H, m) 7.77 (1H, s) |
| 22 | $n_D^{21.0}$ 1.6116 | |
| 23 | | 1.66 (9H, s) 2.30 (3H, s) 3.48 (2H, t) 4.18 (2H, t) 6.77−7.20 (6H, m) 7.77 (1H, s) |
| 24 | $n_D^{21.5}$ 1.5876 | |
| 25 | | 1.66 (9H, s) 2.29 (3H, s) 3.47 (2H, t) 4.17 (2H, t) 6.48 (1H, t) 6.73−7.12 (6H, m) 7.78 (1H, s) |
| 26 | mp 109−113 | |
| 27 | mp 33−36 | |
| 28 | $n_D^{20.6}$ 1.5755 | |
| 29 | mp 111−113 | |
| 30 | | 1.66 (9H, s) 2.30 (3H, s) 4.30 (2H, t) 4.58 (2H, t) 6.73−7.32 (5H, m) 7.85 (1H, s) |
| 31 | mp 102−105 | |
| 33 | mp 98−100 | |
| 34 | $n_D^{23.8}$ 1.5826 | |

Table 1B (cont'd)

| Compound No. | Physical property mp (℃) 又は $n_D$ | PMR δ ppm, $CDCl_3$ |
|---|---|---|
| 35 | $n_D^{21.8}$ 1.5929 | |
| 37 | | 1.65 (9H, s) 2.28 (3H, s) 3.46 (2H, t) 4.18 (2H, t) 6.72-7.38 (6H, m) 7.78 (1H, s) |
| 39 | mp126-130 | |
| 41 | mp109-113 | |
| 42 | mp104-106 | |
| 43 | mp96-99 | |
| 45 | mp109-111 | |
| 46 | | 1.66 (9H, s) 2.27 (3H, s) 2.88 (2H, t) 3.38 (3H, s) 3.46 (2H, t) 3.60 (2H, t) 4.16 (2H, t) 6.71-7.23 (6H, m) 7.78 (1H, s) |
| 47 | | 1.20 (3H, t) 1.65 (9H, s) 2.63 (2H, q) 3.45 (2H, t) 4.20 (2H, t) 6.70-7.40 (6H, t) 7.8 (1H, s) |
| 49 | | 1.20 (2H, t) 1.65 (9H, s) 2.63 (2H, q) 3.40 (2H, t) 4.20 (2H, t) 6.70-7.40 (6H, m) 7.80 (1H, s) |
| 51 | | 1.65 (9H, s) 2.38 (6H, s) 3.45 (2H, t) 4.06 (2H, t) 6.70-7.58 (6H, m) 7.80 (1H, s) |
| 52 | | 1.65 (9H, s) 2.25 (3H, s) 3.75 (2H, t) 4.10 (2H, t) 5.25 (1H, s) 6.70-7.35 (6H, m) 7.62 (1H, s) |
| 53 | mp43-46 | |
| 55 | | 1.20 (3H, t) 1.65 (9H, s) 2.63 (2H, q) 3.75 (2H, t) 4.10 (2H, t) 5.25 (1H, s) 6.70-7.35 (6H, m) 7.62 (1H, s) |
| 57 | mp77-81 | |
| 59 | mp96-99 | |
| 61 | mp132-134 | |
| 62 | mp97-99 | |
| 63 | mp96-98 | |
| 64 | $n_D^{23.3}$ 1.5831 | |
| 65 | $n_D^{25.4}$ 1.5934 | |
| 67 | mp84-86 | |
| 69 | $n_D^{25.4}$ 1.5725 | |
| 71 | $n_D^{25.4}$ 1.5706 | |
| 73 | mp147-150 | |
| 74 | $n_D^{25.4}$ 1.5725 | |
| 75 | | 1.65 (9H, s) 2.26 (3H, s) 3.30 (2H, t) 3.91 (2H, t) 3.99 (2H, s) 6.63-7.29 (11H, m) 7.62 (1H, s) |
| 77 | | 1.33 (6H, d) 1.65 (9H, s) 2.26 (3H, s) 3.46 (2H, t) 4.15 (2H, t) 4.49 (1H, dt) 6.68-6.90 (6H, m) 7.77 (1H, s) |

Table 1B (cont'd)

| Compound No. | Physical property mp (℃) 又は $n_D$ | PMR δ ppm, $CDCl_3$ |
|---|---|---|
| 79 | $n_D^{26.2}$ 1.5899 | |
| 80 | mp147−150 | |
| 81 | $n_D^{26.4}$ 1.5829 | |
| 83 | | 1.65 (9H, s) 2.26 (3H, s) 3.46 (2H, t) 3.80 (3H, s) 4.15 (2H, t) 6.67−6.90 (6H, m) 7.77 (1H, s) |
| 85 | | 1.50 (3H, d) 1.65 (9H, s) 2.26 (3H, s) 4.07 (2H, d) 5.02 (1H, m) 6.72−7.28 (6H, m) 7.88 (1H, s) |
| 86 | | 1.62−1.65 (12H, m) 2.27 (3H, s) 3.97 (3H, m) 6.70−7.27 (6H, m) 7.78 (1H, s) |
| 87 | | 1.65 (9H, s) 2.30 (3H, s) 3.50 (2H, t) 4.20 (2H, t) 6.79−7.59 (6H, m) 7.78 (1H, s) |
| 89 | | 1.46 (9H, s) 2.20 (3H, s) 2.33 (3H, s) 3.41 (3H, s) 4.04 (2H, t) 4.18 (2H, t) 6.79−7.13 (6H, m) |
| 90 | mp186−188 | |
| 92 | $n_D^{26.4}$ 1.5925 | |
| 94 | | 1.03 (9H, s) 1.67 (9H, s) 1.82 (2H, m) 2.27 (3H, s) 3.47 (2H, t) 3.90 (2H, t) 4.15 (2H, t) 6.67−6.93 (6H, m) 7.78 (1H, s) |
| 96 | | 1.25 (3H, t) 1.65 (9H, s) 2.27 (3H, s) 3.47 (2H, t) 3.61 (2H, q) 4.10−4.20 (4H, m) 6.66−6.93 (6H, m) 7.79 (1H, s) |
| 97 | | 1.63 (9H, s) 1.90−2.10 (4H, m) 2.27 (3H, s) 3.15 (2H, t) 3.92 (2H, t) 6.72−7.27 (6H, m) 7.65 (1H, s) |
| 98 | | 0.3−0.4 (2H, m) 0.6−0.7 (2H, m) 1.15−1.35 (1H, m) 1.65 (9H, s) 2.25 (3H, s) 3.29 (2H, d) 4.15 (2H, d) 6.67−6.92 (6H, m) 7.77 (1H, s) |
| 100 | | 1.65 (9H, s) 2.27 (3H, s) 3.47 (2H, m) 4.10−4.29 (4H, m) 4.76 (2H, m) 6.68−6.93 (6H, s) 7.78 (1H, s) |
| 101 | mp160−162 | |
| 102 | | 1.66 (9H, s) 2.27 (3H, s) 3.42 (3H, s) 3.48 (2H, t) 4.18 (2H, t) 4.43 (2H, s) 6.73−7.33 (6H, m) 7.79 (1H, s) |
| 103 | mp91−94 | |
| 104 | | 1.33 (9H, s) 1.66 (9H, s) 2.27 (3H, s) 3.47 (2H, t) 4.16 (2H, t) 6.71−6.95 (6H, m) 7.78 (1H, s) |
| 105 | | 1.66 (9H, s) 2.27 (3H, s) 2.50−2.80 (2H, m) 3.47 (2H, t) 4.10−4.30 (4H, m) 6.65−7.06 (6H, m) 7.77 (1H, s) |
| 106 | | 1.65 (9H, s) 2.29 (3H, s) 3.49 (2H, t) 4.18 (2H, t) 6.62−7.50 (6H, m) 7.78 (1H, s) |
| 108 | | 1.22 (3H, t) 1.65 (9H, s) 2.26 (3H, s) 3.40−4.20 (12H, m) 6.66−6.93 (6H, m) 7.78 (1H, s) |
| 110 | | 1.66 (9H, s) 2.29 (3H, s) 3.48 (2H, t) 4.17 (2H, t) 6.73−7.38 (11H, m) 7.79 (1H, s) |
| 111 | $n_D^{26.2}$ 1.6161 | |
| 112 | $n_D^{26.4}$ 1.5770 | |
| 113 | mp127−128 | |
| 114 | | 1.66 (9H, s) 2.33 (3H, s) 3.50 (2H, t) 4.21 (2H, t) 6.83−7.86 (7H, m) 9.93 (1H, s) |

Table 1B (cont'd)

| Compound No. | Physical property mp (℃) 又は $n_D$ | PMR δppm. $CDCl_3$ |
|---|---|---|
| 115 | mp130−133 | |
| 116 | mp124−127 | |
| 117 | mp163−164 | |
| 118 | $n_D^{27.0}$ 1.5886 | |
| 119 | mp124−127 | |
| 120 | | 1.63 (9H. s) 2.27 (3H. s) 2.34 (3H. s) 3.39 (2H. t) 4.56−4.15 (5H. m) 6.70−7.14 (6H. m) 7.69 (1H. s) |
| 121 | | 1.66 (9H. s) 3.53 (2H. t) 4.30 (2H. t) 7.01 (2H. d) 7.08 (1H. s) 7.62 (2H. s) 7.78 (1H. s) |
| 122 | | 1.65 (9H. s) 3.89 (2H. m) 4.27 (2H. t) 5.27 (1H. t) 7.00 (2H. d) 7.08 (1H. s) 7.60−7.68 (3H. m) |
| 123 | | 1.66 (9H. s) 2.25 (3H. s) 2.88 (2H. t) 3.23 (2H. m) 4.10 (2H. t) 5.30 (1H. s) 6.73−7.20 (6H. m) 7.63 (1H. s) |
| 124 | | 1.65 (9H. s) 3.76 (2H. m) 4.22 (2H. t) 5.30 (1H. t) 6.93−7.37 (6H. m) 7.61 (1H. s) |
| 125 | | 1.66 (9H. s) 2.30 (3H. s) 2.48 (3H. s) 4.29 (2H. s) 4.59 (2H. s) 6.74−7.29 (6H. m) 7.86 (1H. s) |
| 126 | | 1.65 (9H. s) 2.25 (3H. s) 2.48 (3H. s) 3.73 (2H. m) 4.09 (2H. t) 5.27 (1H. t) 6.72−7.30 (6H. m) 7.63 (1H. s) |
| 127 | | 1.66 (9H. s) 2.28 (3H. s) 3.75 (2H. s) 4.12 (2H. t) 5.26 (1H. t) 6.75−7.30 (6H. m) 7.64 (1H. s) |
| 128 | $n_D^{26.2}$ 1.5864 | |
| 129 | | 1.50 (3H. d) 1.66 (9H. s) 2.25 (3H. s) 3.80−4.15 (3H. m) 5.06 (1H. d) 6.71−7.33 (6H. m) 7.65 (1H. s) |
| 130 | | 1.47 (3H: d) 1.63 (9H. s) 2.15 (3H. s) 3.90−4.20 (3H. m) 4.79 (2H. d) 6.74−7.27 (7H. m) 7.65 (1H. s) |
| 131 | | 1.65 (9H. s) 3.43 (2H. t) 4.16 (2H. t) 6.57 (1H. s) 6.94−7.33 (5H. m) 7.46 (1H. s) 7.77 (1H. s) |
| 132 | | 1.65 (9H. s) 2.28 (3H. s) 3.46 (2H. t) 4.18 (2H. t) 6.72−7.38 (6H. m) 7.78 (1H. s) |
| 133 | | 1.27 (3H. t) 2.25 (3H. s) 2.79−2.90 (4H. m) 3.37 (3H. s) 3.61 (2H. t) 3.95 (2H. m) 4.09 (2H. t) 6.15 (1H. t) 6.72−7.27 (6H. m) 8.43 (1H. s) |
| 134 | | 2.30 (3H. s) 4.52 (2H. t) 4.66 (2H. t) 6.70−7.52 (10H. m) 8.14 (1H. d) 8.94 (1H. d) |
| 135 | mp108−111 | |
| 136 | | 1.65 (9H. s) 2.10 (6H. s) 3.45 (2H. t) 4.25 (2H. t) 6.60−7.50 (6H. m) 7.77 (1H. s) |
| 137 | | 1.65 (9H. s) 2.32 (3H. s) 3.48 (2H. t) 4.19 (2H. t) 6.83−7.38 (6H. m) 7.75 (1H. s) |
| Comparative example | mp128−130 | |

In the above Table 1A,

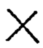 and 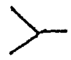

on the heterocyclic group for B refer to tert.-$C_4$-$H_9$ and iso-$C_3H_7$, respectively.

Moreover, in the above Table 1A, the compounds shown under the compounds Nos. 8, 9, 12 - 14, 32, 36, 38, 40, 44, 48, 50, 54, 56, 58, 60, 66, 68, 70, 72, 76, 78, 82, 84, 88, 91, 93, 95, 99, 107, 109, 138 and 139 were listed as compounds which are within the scope of this invention although they were not prepared in the Examples.

Example 13

5 parts by weight of the compound No. 2, 35 parts by weight of bentonite, 57 parts by weight of talc, 1 part by weight of Neopelex Powder (trade name: produced by Kao Atlas) and 2 parts by weight of sodium lignin sulfonate were homogeneously mixed, and then small amount of water was added thereto and the mixture was kneaded, followed by granulation and drying to obtain granules.

Example 14

50 parts by weight of the compound No. 5, 48 parts by weight of kaolin and 2 parts by weight of Neopelex Powder (trade name: produced by Kao Atlas) were homogeneously mixed, and then crushed to obtain wettable powder.

Example 15

20 parts by weight of the compound No. 6, 70 parts by weight of xylene and 10 parts by weight of Toxanon (trade name: produced by Sanyo Kasei Kogyo) were homogeneously mixed and dissolved to obtain emulsion.

Example 16 Activity test against green rice leafhopper

The compounds shown in Table 1 were formulated similarly as described in Example 15, and each formulation was diluted with water containing a surfactant (0.01 %) to 300 ppm to prepare a chemical solution. In each chemical solution was dipped young rice seedlings for 30 seconds, and the rice seedlings after air drying were inserted into a glass cylinder. Ten 4th instar green rice leafhopper larvae were freed into the cylinder, and the cylinder was left to stand stoppered with a porous plug in a thermostatic chamber of 25 °C. 4 days later, the numbers of live and dead insects were counted to determine the % mortality. The results are shown in Table 2.

In Table 2, those with mortality of 100 % are shown as A, those with 99 to 80 % as B, those with 79 to 60 % as C and those with less than 60 % as D.

Table 2

| Compound No. | Activity | Compound No. | Activity |
|---|---|---|---|
| 2 | A | 71 | A |
| 3 | A | 73 | A |
| 4 | A | 74 | A |
| 5 | A | 77 | A |
| 6 | A | 83 | A |
| 7 | A | 85 | A |
| 10 | A | 86 | A |
| 11 | A | 87 | A |
| 17 | A | 94 | A |
| 18 | A | 96 | A |
| 19 | A | 98 | A |
| 20 | A | 100 | A |
| 21 | A | 101 | A |
| 22 | A | 102 | A |
| 23 | A | 103 | A |
| 25 | A | 104 | A |
| 29 | A | 105 | A |
| 30 | A | 106 | A |
| 33 | A | 108 | A |
| 34 | A | 111 | A |
| 37 | A | 112 | A |
| 39 | A | 121 | A |
| 41 | A | 122 | A |
| 42 | A | 123 | A |
| 45 | A | 124 | A |
| 46 | A | 125 | A |
| 47 | A | 126 | A |
| 51 | A | 128 | A |
| 52 | A | 129 | A |
| 55 | A | 132 | A |
| 59 | A | 133 | A |
| 64 | A | 135 | A |
| 65 | A | Comparative example 1 | D |
| 67 | A | | |

Example 17 Activity test against brown rice planthopper

The compounds shown in Table 1 were formulated similarly as described in Example 15, and each formulation was diluted with water containing a surfactant (0.01 %) to 300 ppm to prepare a chemical solution. In each chemical solution was dipped young rice seedlings for 30 seconds, and the rice seedlings after air drying were inserted into a glass cylinder. Ten 3rd instar brown rice planthopper larvae were freed into the cylinder, and the cylinder was left to stand stoppered with a porous plug in a thermostatic chamber of 25 °C. 4 days later, the numbers of live and dead insects were counted to determine the % mortality. The results are shown in Table 3.

In Table 3, those with mortality of 100 % are shown as A, those with 90 to 80 % as B, those with 79 to 60 % as C and those with less and 60 % as D.

Table 3

| Compound No. | Activity | Compound No. | Activity |
|---|---|---|---|
| 4 | A | 73 | B |
| 6 | B | 74 | B |
| 7 | A | 87 | B |
| 17 | A | 94 | A |
| 18 | A | 96 | A |
| 19 | A | 98 | A |
| 20 | B | 100 | A |
| 29 | B | 102 | A |
| 30 | A | 103 | A |
| 37 | A | 105 | A |
| 41 | B | 108 | A |
| 42 | B | 111 | B |
| 45 | A | 120 | B |
| 46 | A | 122 | A |
| 51 | A | 123 | B |
| 52 | B | 124 | A |
| 59 | A | 127 | B |
| 63 | B | 128 | A |
| 64 | B | 129 | A |
| 65 | B | 132 | A |
| 69 | A | Comparative example 1 | D |
| 71 | A | | |

Example 18 Activity test against diamondback moth

The compounds shown in Table 1 were formulated similarly as described in Example 15, and each formulation was diluted with water containing a surfactant (0.01 %) to 300 ppm to prepare a chemical solution. In each chemical solution cabbage leaf strip (5 cm x 5 cm) was dipped for 30 seconds, and placed in a plastic cup. After air drying, ten 3rd instar diamondback moth larvae were freed, and the plastic cup was closed with a lid and left to stand in a thermostatic chamber of 25 °C. 2 days later, the numbers of live and dead insects were counted to determine the % mortality. The results are shown in Table 4.

In Table 4, those with mortality of 100 % are shown as A, those with 99 to 80 % as B, those with 79 to 60 % as C and those with less than 60 % as D.

TABLE 4

| Compound No. | Activity | Compound No. | Activity |
|---|---|---|---|
| 2 | A | 59 | B |
| 3 | B | 62 | B |
| 4 | A | 63 | A |
| 5 | A | 64 | A |
| 7 | A | 65 | A |
| 10 | A | 67 | A |
| 11 | B | 71 | B |
| 17 | A | 73 | A |
| 18 | A | 77 | A |
| 19 | A | 83 | A |
| 20 | A | 94 | A |
| 21 | A | 96 | B |
| 22 | B | 98 | A |
| 23 | A | 100 | A |
| 25 | A | 102 | A |
| 29 | A | 103 | A |
| 30 | A | 105 | A |
| 33 | A | 108 | A |
| 34 | A | 110 | A |
| 37 | A | 111 | A |
| 39 | A | 112 | A |
| 41 | B | 114 | A |
| 42 | B | 116 | A |
| 45 | A | 120 | B |
| 46 | A | 124 | A |
| 47 | A | 128 | A |
| 51 | B | 132 | A |
| 52 | A | 133 | B |
| 53 | A | Comparative example 1 | D |

Example 19 Activity test against female adult citrus red mite

10 female adult citrus red mites were provided for the test of mulberry leaf disks (diameter 20 mm). On the other hand, the compounds shown in Table 1 were formulated similarly as described in Example 15, and each formulation was diluted to 300 ppm with water containing a surfactant (0.01 %) to prepare a chemical solution and 5 ml of the chemical solution was sprayed on leaf disks. The disks was left to stand in a thermostatic chamber of 25 °C, and 3 days later in the numbers of live and dead mites were counted to determine the % mortality. The results are shown in Table 5.

In Table 5, those with mortality of 100 % are shown as A, those with 99 to 80 % as B, those with 79 to 60 % as C and those with less than 60 % as D.

Table 5

| Compound No. | Activity | Compound No. | Activity |
|---|---|---|---|
| 2 | A | 67 | A |
| 3 | A | 77 | A |
| 4 | A | 83 | A |
| 5 | A | 86 | A |
| 6 | A | 87 | A |
| 7 | A | 94 | A |
| 10 | A | 96 | A |
| 17 | A | 98 | A |
| 18 | A | 100 | A |
| 19 | A | 102 | A |
| 20 | A | 103 | A |
| 21 | A | 104 | A |
| 22 | A | 105 | A |
| 23 | A | 106 | A |
| 24 | A | 108 | A |
| 25 | A | 110 | A |
| 29 | A | 111 | A |
| 30 | A | 112 | A |
| 34 | A | 113 | A |
| 37 | A | 114 | A |
| 39 | A | 120 | A |
| 41 | A | 121 | A |
| 42 | A | 122 | A |
| 45 | A | 124 | A |
| 46 | A | 132 | A |
| 47 | A | 133 | A |
| 51 | A | 135 | A |
| 64 | A | Comparative example 1 | D |
| 65 | A | | |

Example 20 Activity test against female adult twospotted spider mite

10 female adult twospotted spider mites were provided for the test on kidney beans leaf disks (diameter 20 mm). On the other hand, the compounds shown in Table 1 were formulated similarly as described in Example 15, and each formulation was diluted to 300 ppm with water containing a surfactant (0.01 %) to prepare a chemical solution, and the leaf disks was dipped in each chemical solution for 10 seconds. The leaf disk was left to stand in a thermostatic chamber of 25 °C, and 3 days later the numbers of live and dead mites were counted to determine the % mortality. The results are shown in Table 6.

In Table 6, those with mortality of 100 % are shown as A, those with 99 to 80 % as B, those with 79 to 60 % as C and those with less than 60 % as D.

Table 6

| Compound No. | Activity | Compound No. | Activity |
|---|---|---|---|
| 2 | A | 67 | A |
| 3 | A | 69 | B |
| 4 | A | 71 | B |
| 5 | A | 77 | A |
| 6 | A | 83 | A |
| 7 | A | 86 | A |
| 10 | A | 87 | A |
| 11 | A | 94 | A |
| 17 | A | 96 | A |
| 18 | A | 98 | A |
| 19 | A | 100 | A |
| 20 | A | 102 | A |
| 21 | A | 103 | A |
| 22 | A | 104 | A |
| 23 | A | 105 | A |
| 24 | A | 106 | A |
| 25 | A | 108 | A |
| 26 | B | 110 | A |
| 29 | A | 111 | A |
| 30 | A | 112 | A |
| 33 | A | 113 | A |
| 34 | A | 114 | A |
| 37 | A | 116 | A |
| 39 | A | 120 | A |
| 41 | A | 121 | A |
| 42 | A | 122 | A |
| 45 | A | 124 | A |
| 46 | A | 125 | B |
| 47 | A | 127 | B |
| 49 | A | 128 | B |
| 51 | A | 129 | B |
| 53 | A | 131 | A |
| 59 | A | 132 | A |
| 61 | A | 133 | A |
| 63 | A | 135 | A |
| 64 | A | 136 | A |
| 65 | A | Comparative example 1 | D |

Example 21 Activity test against cucumber downy mildew

The compound shown in Table 1 were formulated similarly as described in Example 15, and each formulation was diluted to 200 ppm with water containing a surfactant (0.01 %) to prepare a chemical solution, and a sufficient amount of each chemical solution was sprayed on cucumber seedlings. Two days after spraying, spores of cucumber downy mildew was inoculated by spraying, and the seedlings were left to stand in a room of a temperature of 20 °C and a humidity of 100% for one day. Then, the seedlings were transferred into a warm chamber and on the 10th day after inoculation, the lesion are was compared with that of seedlings in the non-treated district. The results are shown in Table 7.

In Table 7, those without lesion are shown as A, those with lesion area of 10 % or less as compared with the non-treated ones as B, those with about 20 % as C and those with greater affliction as D.

Table 7

| Compound No. | Activity |
|---|---|
| 2 | A |
| 3 | A |
| 4 | B |
| Comparative example 1 | D |

According to the present invention, novel diphenyl ether derivatives having excellent insecticidal and acaricidal effect can be provided.

## Claims

1. A diphenyl ether derivative of the formula:

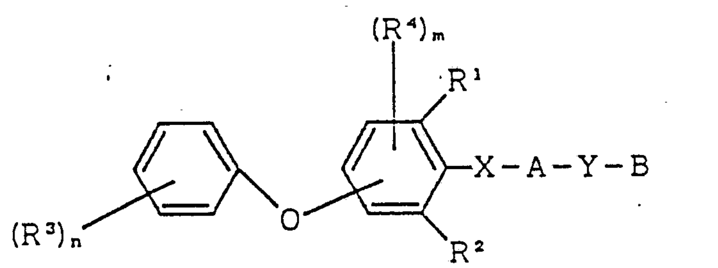

wherein $R^1$, $R^2$, $R^3$ and $R^4$ each independently represent a hydrogen atom, a halogen atom, nitro group, formyl group, hydroxyl group, a lower alkyl group which may be unsubstituted or substituted, a lower alkoxy group which may be unsubstituted or substituted, a lower alkylthio group which may be unsubstituted or substituted or lower alkylsulfonyl group which may be unsubstituted or substituted, an aralkyl group which may be unsubstituted or substituted or an aryloxy group which may be unsubstituted or substituted, with proviso that all of $R^1$, $R^2$, $R^3$ and $R^4$ cannot represent a hydrogen atom at the same time; m is an integer of 1 or 2; n is an integer of 1 to 5; X and Y each independently represent oxygen atom, sulfur atom or an imino group which may be unsubstituted or substituted with a lower alkyl group; A represents a straight or branched alkylene group having 2 to 4 carbon atoms; and B represents a heterocyclic group containing at least one nitrogen atom as the hetero atom.

2. The diphenyl ester derivative according to Claim 1, wherein said lower alkyl group represented by $R^1$, $R^2$, $R^3$ or $R^4$ in the formula (I) is methyl, ethyl, propyl, isopropyl, butyl, isobutyl, sec-butyl, tert-butyl, amyl, isoamyl, sec-amyl, sec-isoamyl (1,2-dimethylpropyl) or tert-amyl (1,1-dimethylpropyl) group; said lower alkoxy group represented by $R^1$, $R^2$, $R^3$ or $R^4$ in the formula (I) is methoxy, ethoxy, propoxy, isopropoxy, butoxy, isobutoxy, sec-butoxy, t-butoxy or amyloxy group; said lower alkylthio group represented by $R^1$, $R^2$, $R^3$ or $R^4$ in the formula (I) is methylthio, ethylthio, propylthio, isopropylthio, butylthio, isobutylthio, sec-butylthio, t-butylthio or amylthio group; said lower alkylsulfonyl group represented by $R^1$, $R^2$, $R^3$ or $R^4$ in the formula (I) is methylsulfonyl, ethylsulfonyl, propylsulfonyl, isopropylsulfonyl, butylsulfonyl, isobutylsulfonyl, sec-butylsulfonyl, t-butylsulfonyl or amylsulfonyl group and said halogen group

represented by $R^1$, $R^2$, $R^3$ or $R^4$ in the formula (I) is fluoride atom, chlorine atom or bromine atom.

3. The diphenyl ether derivative according to Claim 1, wherein said lower alkyl group represented by $R^1$, $R^2$, $R^3$ or $R^4$ in the formula (I); said lower alkoxy group represented by $R^1$, $R^2$, $R^3$, or $R^4$ in the formula (I); said lower alkylthio group represented by $R^1$, $R^2$, $R^3$ or $R^4$ in the formula (I); or said lower alkylsulfonyl group represented by $R^1$, $R^2$, $R^3$ or $R^4$ in the formula (I) is substituted with a halogen atom, a lower alkenyl group, a cycloalkyl group having 3 to 6 carbon atoms, a lower alkylthio group, a lower alkoxy group, a lower alkoxyalkoxy group, a lower alkenyloxy group, a lower alkynyloxy group or an aralkyloxy group.

4. The diphenyl ether derivative according to Claim 3, wherein said lower alkyl group represented by $R^1$, $R^2$, $R^3$ or $R^4$ in the formula (I) is a trifluoromethyl, 2-chloroethyl, 2,2,2-trifluoroethyl, ethoxymethyl, 2-methoxyethyl, 1-ethoxyethyl, 2-ethoxyethyl, 2-allyloxyethyl or 2-propargyloxyethyl group; said lower alkoxy group represented by $R^1$, $R^2$, $R^3$ or $R^4$ in the formula (I) is a trifluoromethoxy, cyclopropylmethoxy, 2-chloroethoxy, 2,2,2-trifluoroethoxy, 2-ethoxyethoxy, 2-benzyloxyethoxy, 2-ethylthioethoxy group; said lower alkylthio group represented by $R^1$, $R^2$, $R^3$ or $R^4$ in the formula (I) is a trifluoromethylthio, 2-chloroethylthio, 2,2,2-trifluoroethylthio, 2-ethoxyethylthio, 2-benzyloxyethylthio or 2-ethylthioethylthio group; and said lower alkylsulfonyl group represented by $R^1$, $R^2$, $R^3$ or $R^4$ in the formula (I) is trifluoromethylsulfonyl, 2-chloroethylsulfonyl, 2,2,2-trifluoroethylsulfonyl, 2-ethoxyethylsulfonyl, 2-benzyloxyethylsulfonyl or 2-ethylthioethylsulfonyl group.

5. The diphenyl ether derivative according to Claim 1 wherein, $R^1$ and $R^2$ in the formula (I) is methyl group or chlorine atom; $R^3$ in the formula (I) is methyl group, 2-fluoroethyl group, fluorine atom, bromine atom, chlorine atom, ethoxy, t-butoxy, difluoromethoxy, trifluoromethoxy, 2-methoxyethyl, 2-ethoxyethoxy, methylthio or formyl groups and is located on the 4-position of the benzene ring; and $R^4$ in the formula (I) is hydrogen atom, methyl group or chlorine atom.

6. The diphenyl ether derivative according to Claim 1, wherein said imino group represented by Y in the formula (I) is substituted with a methyl group.

7. The diphenyl ether derivative according to Claim 7, wherein said heterocyclic group represented by B in the formula (I) is a pyridazinone group.

8. The diphenyl ether derivative according to Claim 1, wherein said derivative is one selected from the group consisting of

2-tert-butyl-4-chloro-5-{2-[2,6-dimethyl-4-(4-chlorophenoxy)phenoxy]ethylthio}-2(2H)pyridazinone,
2-tert-butyl-4-chloro-5-{2-[2,6-dimethyl-4-(4-methylphenoxy)phenoxy]ethylthio}-3(2H)pyridazinone,
2-tert-butyl-4-chloro-5-{2-[2,6-dimethyl-4-(4-fluorophenoxy)phenoxy]ethylamino}-3(2H)pyridazinone,
5-chloro-6-ethyl-4-{2,6-dimethyl-4-(4-fluorophenoxy)-phenoxy]ethylamino}pyrimidine,
2-tert-butyl-4-chloro-5-{2-[2,6-dimethyl-4-(4-trifluoromethylphenoxy)phenoxy]ethylthio}-3(2H)pyridazinone,
2-tert-butyl-4-chloro-5-{2-[2-chloro-6-methyl-4-(4-chlorophenoxy)phenoxy]ethylamino}-3(2H)pyridazinone,
2-tert-butyl-4-chloro-5-{2-[2-chloro-6-methyl-4-(4-trifluoromethoxyphenoxy)phenoxy]ethylthio}-3(2H)-pyridazinone,
2-tert-butyl-4-chloro-5-{2-[2-chloro-6-methyl-4-(4-i-propoxyphenoxy)phenoxy]ethylthio}-3(2H)pyridazinone,
2-tert-butyl-4-chloro-5-{2-[2-chloro-6-methyl-4-(4-t-butoxyphenoxy)phenoxy]ethylthio}-3(2H)pyridazinone,
2-tert-butyl-4-chloro-5-{2-[2-chloro-6-methyl-4-(4-formylphenoxy)phenoxy]ethylthio}-3(2H)pyridazinone,
2-tert-butyl-4-chloro-5-{2-[2-chloro-6-methyl-4-(4-difluoromethylphenoxy)phenoxy]ethylthio}3(2H)pyridazinone and
2-tert-butyl-4-chloro-5-{2-[2-chloro-6-methyl-4-(4-(2-ethoxyethyl)phenoxy)phenoxy]ethylthio}3(2H)pyridazinone.

9. A process for preparing a diphenyl ether derivative of the formula:

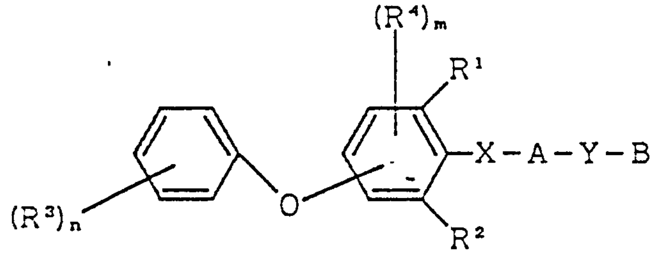

wherein $R^1$, $R^2$, $R^3$ and $R^4$ each independently represent hydrogen atom, a halogen atom, nitro group, formyl group, hydroxyl group, a lower alkyl group which may be unsubstituted or substituted, a lower alkoxy group which may be unsubstituted or substituted, a lower alkylthio group which may be

unsubstituted or substituted or lower alkylsulfonyl group which may be unsubstituted or substituted, an aralkyl group which may be unsubstituted or substituted or an aryloxy group which may be unsubstituted or substituted, with proviso that all of $R^1$, $R^2$, $R^3$ and $R^4$ cannot represent a hydrogen atom at the same time; m is an integer of 1 or 2; n is an integer of 1 to 5; X and Y each independently represent oxygen atom, sulfur atom or an imino group which may be unsubstituted or substituted with a lower alkyl group; A represents a straight or branched alkylene group having 2 to 4 carbon atoms; and B represents a heterocyclic group containing at least one nitrogen atom as the hetero atom,
which comprises allowing a compound of the formula:
B-Hal
wherein B represents a heterocyclic group containing at least one nitrogen atom as the hetero atom; Hal represents a halogen atom,
to react with a compound of the formula:

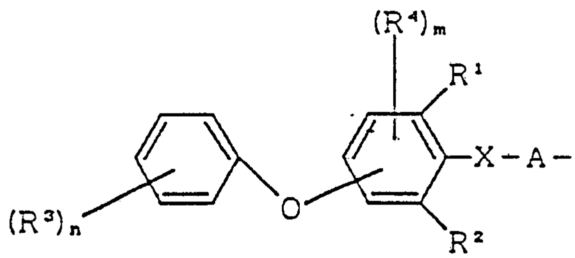

(III)

wherein $R^1$, $R^2$, $R^3$, $R^4$, m, n, X, Y and A are the same as defined above.
10. A process for producing a diphenyl ether derivative of the formula:

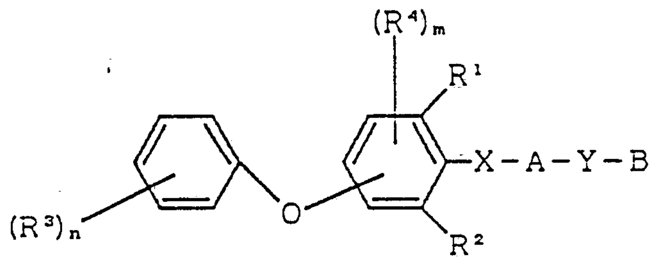

wherein $R^1$, $R^2$, $R^3$ and $R^4$ each independently represent a hydrogen atom, a halogen atom, a nitro group, a formyl group, a hydroxyl group, a lower alkyl group which may be unsubstituted or substituted, a lower alkoxy group which may be unsubstituted or substituted, a lower alkylthio group which may be unsubstituted or substituted or lower alkylsulfonyl group which may be unsubstituted or substituted, an aralkyl group which may be unsubstituted or substituted or an aryloxy group which may be unsubstituted or substituted, with proviso that all of $R^1$, $R^2$, $R^3$ and $R^4$ cannot represent a hydrogen atom at the same time; m is an integer of 1 or 2; n is an integer of 1 to 5; X and Y each independently represent oxygen atom, sulfur atom or an imino group which may be unsubstituted or substituted with a lower alkyl group; A represents a straight or branched alkylene group having 2 to 4 carbon atoms; and B represents a heterocyclic group containing at least one nitrogen atom as the hetero atom,
which comprises allowing a compound of the formula:
B-Y-H
wherein B and Y are the same as defined above,
to react with a compound of the formula:

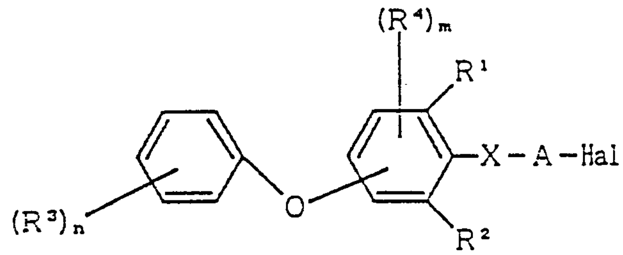

wherein $R^1$, $R^2$, $R^3$, $R^4$, m, n, X and A are the same as defined above.

11. A process for preparing a diphenyl ether derivative of the formula:

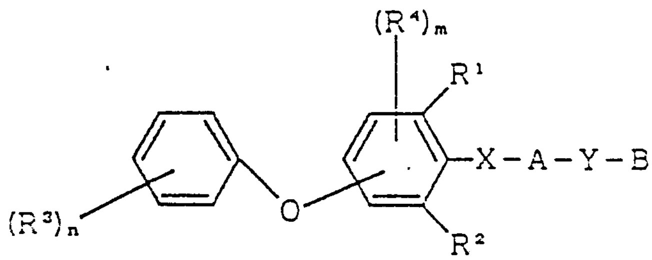

wherein $R^1$, $R^2$ and $R^4$ each independently represent a hydrogen atom, a halogen atom, a nitro group, a formyl group, a hydroxyl group, a lower alkyl group which may be unsubstituted or substituted, a lower alkoxy group which may be unsubstituted or substituted, a lower alkylthio group which may be unsubstituted, a lower alkylthio group which may be unsubstituted or substituted or lower alkylsulfonyl group which may be unsubstituted or substituted, an aralkyl group which may be unsubstituted or substituted or an aryloxy group which may be unsubstituted or substituted, $R^3$ is a group of the formula:

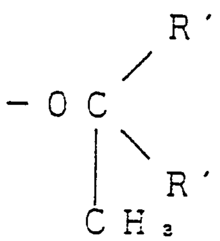

wherein R′ represents a lower alkyl group;
m is an integer of 1 or 2; n is an integer of 1 to 5; X and Y each independently represent oxygen atom, sulfur atom or an imino group which may be unsubstituted or substituted with a lower alkyl group; A represents a straight or branched alkylene group having 2 to 4 carbon atoms; and B represents a heterocyclic group containing at least one nitrogen atom as the hetero atom,
which comprises allowing a compound of the formula:

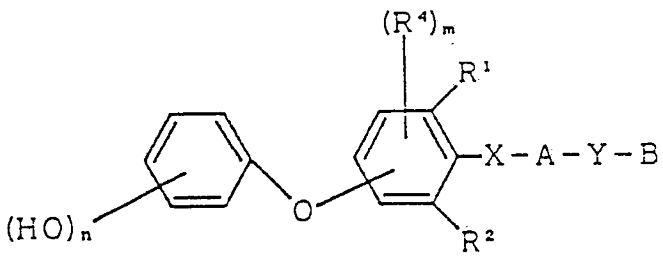

wherein $R^1$, $R^2$, $R^4$, m, n, X, Y, A and B are the same as defined above, to react with a compound of the formula:

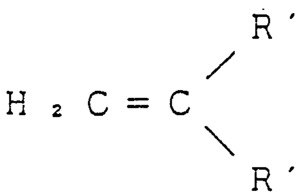

wherein R′ is the same as defined above.

12. A process for producing a diphenyl ether derivative of the formula;

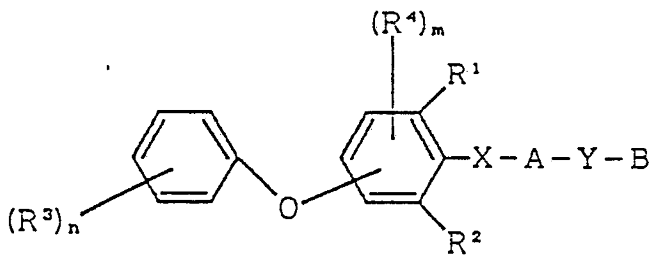

wherein $R^1$, $R^2$, and $R^4$ each independently represent a hydrogen atom, a halogen atom, a nitro group, a formyl group, a hydroxyl group, a lower alkyl group which may be unsubstituted or substituted, a lower alkoxy group which may be unsubstituted or substituted, a lower alkylthio group which may be unsubstituted or substituted or lower alkylsulfonyl group which may be unsubstituted or substituted, an aralkyl group which may be unsubstituted or substituted or an aryloxy group which may be unsubstituted or substituted, $R^3$ is a difluoromethyl group; m is an integer of 1 or 2; n is an integer of 1 to 5; X and Y each independently represent oxygen atom, sulfur atom or an imino group which may be unsubstituted or substituted with a lower alkyl group; A represents a straight or branched alkylene group having 2 to 4 carbon atoms; and B represents a heterocyclic group containing at least one nitrogen atom as the hetero atom,
which comprises allowing a compound of the formula:

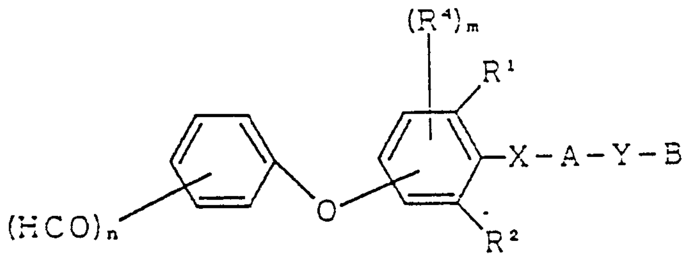

wherein $R^1$, $R^2$, $R^4$, m, n, X,, Y, A and B are the same as defined above, to react with diethylaminosulfur trifluoride.

13. A process for producing a diphenyl ether derivative of the formula:

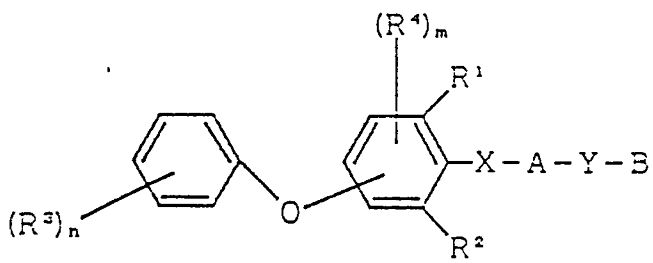

wherein $R^1$, $R^2$ and $R^4$ each independently represent a a hydrogen atom, a halogen atom, a nitro group, a formyl group, a hydroxyl group, a lower alkyl group which may be unsubstituted or substituted, a lower alkoxy group which may be unsubstituted or substituted, a lower alkylthio group which may be unsubstituted or substituted or lower alkylsulfonyl group which may be unsubstituted or substituted, an aralkyl group which may be unsubstituted or substituted or an aryloxy group which may be unsubstituted or substituted, $R^3$ is a trifluoromethoxy group; m is an integer of 1 or 2; n is an integer of 1 to 5; X and Y each independently represent oxygen atom, sulfur atom or an imino group which may be unsubstituted or substituted with a lower alkyl group; A represents a straight or branched alkylene group having 2 to 4 carbon atoms; and B represents a heterocyclic group containing at least one nitrogen atom as the hetero atom,
which comprises allowing a compound of the formula:

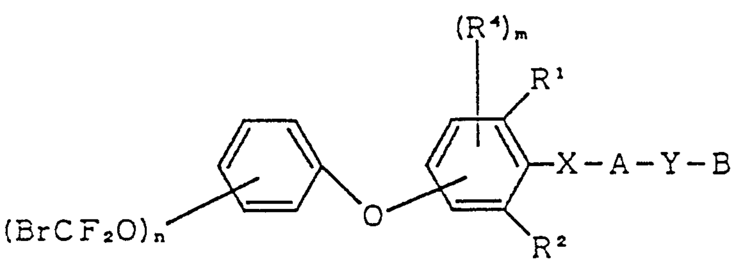

wherein $R^1$, $R^2$, $R^4$, m, n, X, Y, A and B are the same as defined above, to react with $AgBF_4$.

14. The process according to Claim 9, 10 or 11 wherein said reaction is carried out in the presence of a base.

15. The process according to Claim 14, wherein said base is selected from the group consisting of triethylamine, pyridine, N,N-dimethylaniline, sodium methoxide, sodium ethoxide, sodium hydride, sodium amide, sodium hydroxide, potassium hydroxide, sodium carbonate, sodium hydrogen carbonate and potassium carbonate.

16. The process according to Claim 9, 10, 11, 12 or 13, wherein said reaction is carried out in the presence of a solvent.

17. The process accoridng to Claim 16, wherein said solvent is selected from the group consisting of benzene, toluene, xylene, methyl naphthalene, petroleum ether, ligroin, hexane, chlorobenzene, dichlorobenzene, methylene chloride, chloroform, dichloroethane, trichloroethylene, cyclohexane, diethyl ether, ethylene glycol dimethyl ether, tetrahydrofuran, dioxane, acetone, methyl ethyl ketone, methyl isobutyl ketone, methanol, ethanol, ethylene glycol or hydrates thereof, N,N-dimethylformamide, N,N-dimethylacetamide, 1,3-dimethyl-2-imidazolidinone, dimethylsulfoxide, water and mixtures of the above solvents.

18. The process according to Claim 9, 10 11, 12 or 13, wherein the reaction is carried out in the range from 0 °C to the boiling point of the solvent employed.

19. The process according to Claim 11, wherein the reaction is carried out in the presence of an acid catalyst selected form the group consisting of sulfuric acid, phosphoric acid, polyphosphoric acid, hydrogen chloride, zinc chloride, titanium tetrachloride and aluminum chloride.

20. An insecticide containing a diphenyl ether derivative of the formula:

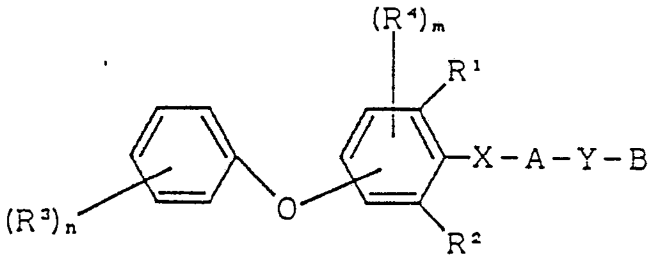

wherein $R^1$, $R^2$, $R^3$ and $R^4$ each independently represent a hydrogen atom, a halogen atom, a nitro group, a formyl group, a hydroxyl group, a lower alkyl group which may be unsubstituted or substituted, a lower alkoxy group which may be unsubstituted or substituted, a lower alkylthio group which may be unsubstituted or substituted or lower alkylsulfonyl group which may be unsubstituted or substituted, an aralkyl group which may be unsubstituted or substituted or an aryloxy group which may be unsubstituted or substituted, with proviso that all of $R^1$, $R^2$, $R^3$ and $R^4$ cannot represent a hydrogen atom at the same time; m is an integer of 1 or 2; n is an integer of 1 to 5; X and Y each independently represent oxygen atom, sulfur atom or an imino group which may be unsubstituted or substituted with a lower alkyl group; A represents a straight or branched alkylene group having 2 to 4 carbon atoms; and B represents a heterocyclic group containing at least one nitrogen atom as the hetero atom.

21. An acaricide containing as the active ingredient a diphenyl ether derivative of the formula:

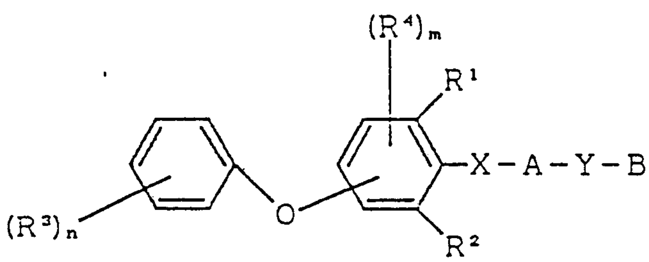

wherein $R^1$, $R^2$, $R^3$ and $R^4$ each independently represent a hydrogen atom, a halogen atom, a nitro group, a formyl group, a hydroxyl group, a lower alkyl group which may be unsubstituted or substituted, a lower alkoxy group which may be unsubstituted or substituted, a lower alkylthio group which may be unsubstituted or substituted or lower alkylsulfonyl group which may be unsubstituted or substituted, an aralkyl group which may be unsubstituted or substituted or an aryloxy group which may be unsubstituted or substituted, with proviso that all of $R^1$, $R^2$, $R^3$ and $R^4$ cannot represent a hydrogen atom at the same time; m is an integer of 1 or 2; n is an integer of 1 to 5; X and Y each independently represent oxygen atom, sulfur atom or an imino group which may be unsubstituted or substituted with a lower alkyl group; A represents a straight or branched alkylene group having 2 to 4 carbon atoms; and B represents a heterocyclic group containing at least one nitrogen atom as the hetero atom.